(19) Europäisches Patentamt
European Patent Office
Office européen des brevets

(11) **EP 4 316 490 A1**

(12) **EUROPEAN PATENT APPLICATION**
published in accordance with Art. 153(4) EPC


(43) Date of publication:
**07.02.2024 Bulletin 2024/06**

(21) Application number: **22774164.2**

(22) Date of filing: **21.03.2022**

(51) International Patent Classification (IPC):
***A61K 31/506*** $^{(2006.01)}$ ***C07D 487/08*** $^{(2006.01)}$
***A61P 35/00*** $^{(2006.01)}$

(52) Cooperative Patent Classification (CPC):
**A61K 31/506; A61P 35/00; C07D 487/08**

(86) International application number:
**PCT/CN2022/081890**

(87) International publication number:
**WO 2022/199503 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.03.2021 CN 202110316525**
**28.01.2022 CN 202210109766**
**28.01.2022 PCT/CN2022/074619**



(71) Applicant: **Sichuan Kelun-Biotech Biopharmaceutical Co., Ltd.**
**Chengdu, Sichuan 611138 (CN)**

(72) Inventors:
- **CHEN, Zhonghui**
**Chengdu, Sichuan 611138 (CN)**
- **YUAN, Xiaoxi**
**Chengdu, Sichuan 611138 (CN)**
- **LEI, Dongmei**
**Chengdu, Sichuan 611138 (CN)**
- **HAN, Runfeng**
**Chengdu, Sichuan 611138 (CN)**
- **HAN, Xiaojun**
**Chengdu, Sichuan 611138 (CN)**
- **TIAN, Qiang**
**Chengdu, Sichuan 611138 (CN)**
- **SONG, Hongmei**
**Chengdu, Sichuan 611138 (CN)**
- **WANG, Jingyi**
**Chengdu, Sichuan 611138 (CN)**

(74) Representative: **Carpmaels & Ransford LLP**
**One Southampton Row**
**London WC1B 5HA (GB)**


(54) **USE OF HETEROCYCLIC COMPOUND IN TREATING DISEASES RELATED TO KINASE DRUG-RESISTANT MUTATION AND METHOD THEREFOR**

(57)

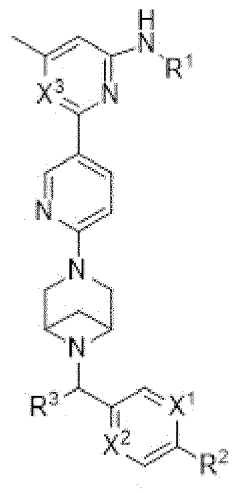

Formula I

EP 4 316 490 A1

The present invention relates to a use of a heterocyclic compound represented by formula I in the preparation of a drug for treating or assisting the treatment of diseases or conditions in an individual relating to a rearranged during transfection (RET) drug-resistant mutation and a treatment method using said heterocyclic compound.

## Description

### Technical Field

The present disclosure relates to the use of a heterocyclic compound in the preparation of a drug for the treatment or adjuvant treatment of a disease or condition associated with an RET (Rearranged during transfection) drug-resistant mutation in an individual and a treatment method using the heterocyclic compound.

### Background

Protein kinases are a class of enzymes catalysing protein phosphorylation reactions. By mediating the process of cell signal transduction, protein phosphorylation regulates the physiological activities of cells, such as cell survival, proliferation, differentiation, apoptosis, and metabolism. The dysfunction of the protein kinases is closely associated with many diseases, including tumours, autoimmune diseases, inflammatory reactions, central nervous system diseases, cardiovascular diseases, diabetes, and the like.

As a protooncogene, RET encodes an RET protein which is a transmembrane receptor type tyrosine protein kinase consisting of a cysteine-rich cadherin-like extracellular domain (for binding to ligands), a transmembrane domain, and an intracellular domain with tyrosine kinase activity. The activated RET protein can activate multiple downstream signal pathways, including RAS/RAF/ERK pathway, PI3K/Akt pathway, and JNK pathway, thereby resulting in cell proliferation, migration, and differentiation. The alteration (mutation or fusion) of the RET gene and the abnormal expression of wild-type RET gene lead to abnormal activation of RET proteins, such that the signal pathways are overactive, which is one of the main mechanisms of carcinogenesis. Abnormally activated RET proteins are involved in the proliferation and invasion of different tumour cells through a variety of signal pathways, thereby affecting the occurrence and development of tumours. The alteration of the RET gene has a more significant effect on downstream cascade reactions, where the mutation of the RET gene is mainly associated with medullary thyroid cancer and papillary thyroid cancer, and the fusion of the RET gene is mainly associated with non-small cell lung cancer and chronic myeloid leukaemia. Therefore, it is of great medical value to inhibit RET activity (Nature Reviews Cancer, 2014, 14 (3): 173-186).

RET inhibitors have great potential for the treatment and prevention of a variety of diseases (such as tumours, irritable bowel syndrome, and the like). At present, two compounds (Selpercatinib (also known as LOXO-292) and Pralsetinib (also known as BLU-667)) have been approved for marketing, and multiple compounds are in clinical trials. However, clinical studies have shown that long-term administration of RET inhibitors can lead to RET drug-resistant mutations (such as G810R, G810S, G810C, Y806C, Y806N, and V738A), which can easily lead to decreased efficacy, relapse, and poor prognosis (Journal of Thoracic Oncology, 2020, 15(4), 541-549; Annals of oncology: official journal of the European Society for Medical Oncology, 2021, 32(2), 261-268; Annals of oncology: official journal of the European Society for Medical Oncology, 2020, 31(12), 1599-1600; Annals of oncology: Official Journal of the European Society for Medical Oncology, 2020, 31(12), 1725-1733, etc.). Therefore, the development of compounds effective for diseases related to RET drug-resistant mutations has a good application prospect.

### Summary

In one aspect, the present disclosure provides the use of a compound of Formula I, a stereoisomer, tautomer, or mixture of stereoisomer and tautomer thereof, an N-oxide thereof, a pharmaceutically acceptable salt, eutecticum, polymorph, or solvate thereof, or a stable isotope derivative, metabolite, or prodrug thereof in the preparation of a drug for the treatment or adjuvant treatment of a disease or condition associated with an RET drug-resistant mutation (e.g., one or more mutations selected from the group consisting of G810R, G810S, G810C, Y806C, Y806N, and V738A) in an individual:

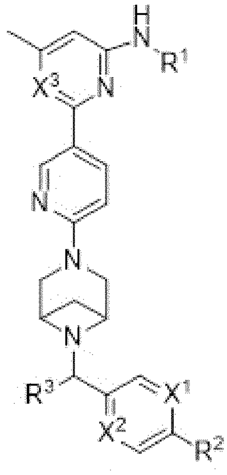

Formula I

wherein:

$R^1$ is selected from 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and

$X^1$, $X^2$ and $X^3$ are each independently selected from CH and N.

In some preferred embodiments, the present disclosure provides the use of a compound 1, a stereoisomer, tautomer, or mixture of stereoisomer and tautomer thereof, an N-oxide thereof, a pharmaceutically acceptable salt, eutecticum, polymorph, or solvate thereof, or a stable isotope derivative, metabolite, or prodrug thereof in the preparation of a medicine for the treatment or adjuvant treatment of a disease or condition associated with an RET drug-resistant mutation (e.g., one or more mutations selected from the group consisting of G810R, G810S, G810C, Y806C, Y806N, and V738A) in an individual, wherein the compound 1 has the following structure:

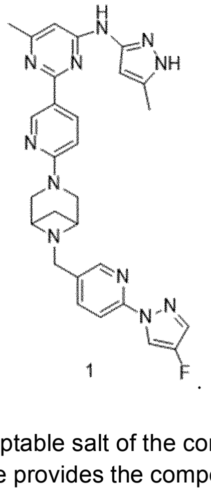

1

For example, the pharmaceutically acceptable salt of the compound 1 is a fumarate of the compound 1.

In another aspect, the present disclosure provides the compound of Formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof for use in the treatment or adjuvant treatment of a disease or condition associated with an RET drug-resistant mutation (e.g., one or more mutations

selected from the group consisting of G810R, G810S, G810C, Y806C, Y806N, and V738A) in an individual.

In another aspect, the present disclosure provides a method for the treatment or adjuvant treatment of a disease or condition associated with an RET drug-resistant mutation (e.g., one or more mutations selected from the group consisting of G810R, G810S, G810C, Y806C, Y806N, and V738A) in an individual, wherein the method comprises administering to the individual an effective amount of the compound of Formula I, the stereoisomer, tautomer, or mixture of stereoisomer and tautomer thereof, the N-oxide thereof, the pharmaceutically acceptable salt, eutecticum, polymorph or solvate thereof, or the stable isotope derivative, metabolite or prodrug thereof.

In another aspect, the present disclosure provides the use of the compound of Formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof in the preparation of a drug for the modulation (e.g., reduction or inhibition) of abnormal RET activity associated with an RET drug-resistant mutation (e.g., one or more mutations selected from the group consisting of G810R, G810S, G810C, Y806C, Y806N, and V738A).

In another aspect, the present disclosure provides the compound of formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof for the modulation (e.g., reduction or inhibition) of abnormal RET activity associated with an RET drug-resistant mutation (e.g., one or more mutations selected from the group consisting of G810R, G810S, G810C, Y806C, Y806N, and V738A).

In another aspect, the present disclosure provides a method for the modulation (e.g., reduction or inhibition) of abnormal RET activity associated with an RET drug-resistant mutation (e.g., one or more mutations selected from the group consisting of G810R, G810S, G810C, Y806C, Y806N, and V738A), wherein the method comprises administrating an effective amount of the compound of Formula I, the stereoisomer, tautomer, or mixture of stereoisomer and tautomer thereof, the N-oxide thereof, the pharmaceutically acceptable salt, eutecticum, polymorph or solvate thereof, or the stable isotope derivative, metabolite or prodrug thereof.

The compound has good inhibition effect on RET drug-resistant mutations, and features good pharmacokinetic, safety and other properties.

## Brief Description of the Drawings

FIG. 1: In vivo efficacy of Compound 1-A and control compound in Ba/F3 KIF5B-RET$^{G810R}$ subcutaneous xenograft tumour model.

## Detailed Description of the Invention

### Definitions

Unless otherwise defined in the context, all technical terms and scientific terms used herein are intended to have the same meaning as commonly understood by those skilled in the art. The reference to a technology used herein is intended to refer to a technology generally understood in the art, including those technological alterations or equivalent technological replacements that are obvious to those skilled in the art. While it is believed that the following terms are well understood by those skilled in the art, the following definitions are still set forth to better explain the present disclosure.

The term "including," "comprising," "having," "containing," or "relating to" and additional variations thereof herein are inclusive or open-ended, and do not exclude additional unlisted elements or method steps, although additional unlisted elements or method steps do not necessarily exist (i.e., these terms also encompass the terms "substantially consisting of" and "consisting of").

As used herein, the term "alkyl" is defined as a linear or branched saturated aliphatic hydrocarbon. In some embodiments, alkyl has from 1 to 12, e.g., from 1 to 6, carbon atoms. For example, as used herein, the terms "$C_{1-6}$ alkyl" and "$C_{1-4}$ alkyl" refer to a linear or branched group having from 1 to 6 carbon atoms and a linear or branched radical having from 1 to 4 carbon atoms respectively (e.g., methyl, ethyl, n-propyl, isopropyl, n-butyl, isobutyl, sec-butyl, tert-butyl, n-pentyl, isopentyl, neopentyl, or n-hexyl), which are optionally substituted with one or more (e.g., from 1 to 3) suitable substituents, e.g., halogen (in this case, the radical is termed "haloalkyl") (e.g., $CH_2F$, $CHF_2$, $CF_3$, $CCl_3$, $C_2F_5$, $C_2Cl_5$, $CH_2CF_3$, $CH_2Cl$, or $-CH_2CH_2CF_3$). The term "$C_{1-4}$ alkyl" refers to a linear or branched aliphatic hydrocarbon chain having from 1 to 4 carbon atoms (i.e., methyl, ethyl, n-propyl, isopropyl, n-butyl, isobutyl, sec-butyl, or tert-butyl).

As used herein, the term "heteroalkyl" refers to an optionally substituted alkyl radical that has one or more backbone chain atoms selected from atoms other than carbon, such as oxygen, nitrogen, sulphur, phosphorus, or combinations thereof. A numerical range (e.g., $C_{1-6}$ heteroalkyl) that may be given refers to the number of carbons in a chain, including from 1 to 6 carbon atoms in this example. For example, $-CH_2OCH_2CH_3$ group is termed $C_3$ heteroalkyl. Its attachment to the rest of the molecule may be through a heteroatom or carbon atom in the heteroalkyl chain.

As used herein, the term "haloalkyl" refers to an alkyl radical substituted with one or more (e.g., from 1 to 3) same or different halogen atoms, and the term "$C_{1-6}$ haloalkyl" and "$C_{1-4}$ haloalkyl" refer to a haloalkyl radical having from 1 to 6 carbon atoms and a haloalkyl radical having from 1 to 4 carbon atoms respectively, such as $-CF_3$, $-C_2F_5$, $-CHF_2$, $-CH_2F$, $-CH_2CF_3$, $-CH_2Cl$, or $-CH_2CH_2CF_3$.

As used herein, the term "hydroxyalkyl" refers to a group formed by substituting hydrogen atom(s) in an alkyl radical with one or more hydroxy, e.g., $C_{1-4}$ hydroxyalkyl or $C_{1-3}$ hydroxyalkyl, and its examples include, but are not limited to, hydroxymethyl, hydroxyethyl, hydroxypropyl, hydroxybutyl, $-CH(OH)CH_3$, $-C(CH_3)_2OH$, and the like.

As used herein, the term "alkoxy" refers to a group formed by inserting an oxygen atom into any reasonable position of an alkyl radical (as defined above), and is, for example, $C_{1-6}$ alkoxy, $C_{1-4}$ alkoxy, or $C_{1-3}$ alkoxy. Representative examples of $C_{1-6}$ alkoxy include, but are not limited to, methoxy, ethoxy, propoxy, isopropoxy, n-propoxy, isopropoxy, n-butoxy, isobutoxy, tert-butoxy, pentyloxy, hexyloxy, $-CH_2-OCH_3$, and the like, and the alkoxy is optionally substituted with one or more (e.g., from 1 to 3) same or different substituents.

As used herein, the term "condensed ring" or "fused ring" refers to a ring system formed by two or more ring structures sharing two adjacent atoms with each other.

As used herein, the term "spiro ring" refers to a ring system formed by two or more ring structures sharing one ring atom with each other.

As used herein, the term "bridged ring" refers to a ring system formed by two or more ring structures sharing two atoms (that are not directly connected) with each other.

As used herein, the term "cycloalkyl" refers to a saturated or unsaturated non-aromatic monocyclic or multicyclic (such as bicyclic) hydrocarbon ring radical, including but not limited to monocycloalkyl (such as cyclopropyl, cyclobutyl, cyclopentyl, cyclohexyl, cycloheptyl, cyclooctyl, and cyclononyl) and bicycloalkyl, including a spiro ring, condensed (fused) ring, or bridged ring system (i.e., spirocycloalkyl, condensed (fused) cycloalkyl, and bridged cycloalkyl, such as bicyclo[1.1.1]pentyl, and bicyclo[2.2.1]heptyl). In the present disclosure, a cycloalkyl radical is optionally substituted with one or more (e.g., from 1 to 3) same or different substituents. A carbon atom on a cycloalkyl radical is optionally substituted with an oxo group (i.e., forming C=O). The term "$C_{3-6}$ cycloalkyl" refers to a cycloalkyl radical having from 3 to 6 ring-forming carbon atoms, which may be a monocycloalkyl radical, such as cyclopropyl, cyclobutyl, cyclopentyl, cyclohexyl, cycloheptyl, or cyclooctyl, and may also be a bicycloalkyl radical, such as $C_{5-8}$ spirocycloalkyl, $C_{5-8}$ bridged cycloalkyl, $C_{5-8}$ fused cycloalkyl, $C_{5-6}$ spirocycloalkyl, $C_{5-6}$ bridged cycloalkyl, or $C_{5-6}$ fused cycloalkyl.

As used herein, the term "cycloalkoxy" means -O-cycloalkyl, where the cycloalkyl is as defined above. Representative examples of cycloalkoxy radicals include, but are not limited to, cyclopropoxy, cyclobutoxy, cyclopentoxy, cyclohexoxy, and the like.

As used herein, the term "heterocyclyl" or "heterocyclic ring" refers to a monocyclic or multicyclic (for example, condensed, spiro, or bridged cyclic) radical having 2 or more than 2 (e.g., 3, 4, 5, 6, 7, 8, 9, 10, 11, 12, 13, or 14) carbon atoms, and one or more (for example, 1, 2, 3, or 4) heteroatoms, wherein the heteroatoms include, but are not limited to, an oxygen atom, a nitrogen atom, and a sulphur atom, and the carbon atoms and heteroatoms on the heterocyclyl are optionally substituted with an oxo group (for example, forming C=O, S(=O), or $S(=O)_2$).

As used herein, the term "4-10-membered heterocyclyl" means a heterocyclyl radical containing from 4 to 10 ring atoms, including but not limited to, 4-9-membered heterocyclyl, 4-8-membered heterocyclyl, 4-7-membered heterocyclyl, 5-6-membered heterocyclyl, 3-8-membered heterocyclyl, 3-7-membered heterocyclyl, 4-7-membered nitrogen-containing heterocyclyl, 4-7-membered oxygen-containing heterocyclyl, 4-7-membered sulphur-containing heterocyclyl, 5-6-membered nitrogen-containing heterocyclyl, 5-6-membered oxygen-containing heterocyclyl, 5-6-membered sulphur-containing heterocyclyl, and the like. The "nitrogen-containing heterocyclyl," "oxygen-containing heterocyclyl," and "sulphur-containing heterocyclyl" each optionally further contain one or more additional heteroatoms selected from oxygen, nitrogen, and sulphur. Examples of 4-10-membered heterocyclyl include, but are not limited to, oxiranyl, aziridinyl, azacyclobutyl, oxacyclobutyl, tetrahydrofuranyl, pyrrolidinyl, pyrrolidonyl (e.g.,

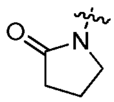

), imidazolidinyl, pyrazolidinyl, tetrahydropyranyl, piperidinyl, morpholinyl, dithianyl, thiomorpholinyl, piperazinyl, and trithianyl.

As used herein, the term "heteroaryl" or "heteroaromatic ring" refers to a monocyclic or multicyclic aromatic group comprising one or more same or different heteroatoms, including a monocyclic heteroaryl radical and a bicyclic or multicyclic ring system comprising at least one heteroaromatic ring (an aromatic ring system comprising at least one heteroatom), which may have 5, 6, 7, 8, 9, 10, 11, 12, 13, or 14 ring atoms, for example, 5, 6, 7, 8, 9, or 10 ring atoms. The heteroatom may be oxygen, nitrogen, or sulphur. A carbon atom and a heteroatom on the heteroaryl are optionally

substituted with an oxo group (for example, forming C=O, S(=O), or $S(=O)_2$).

As used herein, the term "5-10-membered heteroaryl" or "5-10-membered heteroaromatic ring" means a heteroaryl radical (heteroaromatic ring) comprising from 5 to 10 (e.g., from 5 to 6) ring atoms, including 5-10-membered nitrogen-containing heteroaryl, 5-10-member oxygen-containing heteroaryl, 5-10-membered sulphur-containing heteroaryl, 5-6-membered nitrogen-containing heteroaryl, 5-6-membered oxygen-containing heteroaryl, 5-6-membered sulphur-containing heteroaryl, and the like. The "nitrogen-containing heteroaryl," "oxygen-containing heteroaryl," and "sulphur-containing heteroaryl" each optionally comprise one or more additional heteroatoms selected from oxygen, nitrogen, and sulphur. Examples thereof include, but are not limited to, thienyl, furanyl, pyrrolyl, oxazolyl, thiazolyl, imidazolyl, pyrazolyl, isoxazolyl, isothiazolyl, triazolyl, tetrazolyl, oxadiazolyl, thiadiazolyl, etc., or pyridyl, pyridazinyl, pyrimidinyl, pyrazinyl, triazinyl, etc., and a 5-10-membered condensed ring group comprising these radicals.

As used herein, the term "heteroaryl" encompasses a condensed ring structure, and the point of attachment of the condensed ring structure to additional radicals may be on any ring of the condensed ring structure. Therefore, heteroaryl radicals of the present disclosure further include, but are not limited to, (mono)heteroaryl-(mono)heteroaryl, (mono)heteroaryl-(monocyclo)aryl, (mono)heteroaryl-(mono)heterocyclyl, and (mono)heteroaryl-(mono)cycloalkyl, such as 5-6-membered (mono)heteroaryl-5-6-membered (mono)heteroaryl, 5-6 membered (mono)heteroaryl-phenyl, 5-6-membered (mono)heteroaryl-5-6-membered (mono)heterocyclyl, or 5-6-membered (mono)heteroaryl-$C_{4-6}$(mono)cycloalkyl (e.g., 5-6-membered heteroaryl-cyclobutyl, 5-6-membered heteroaryl-cyclopentyl, or 5-6-membered heteroaryl-cyclohexyl). Examples of heteroaryl include, but are not limited to, indolyl, isoindolyl, indazolyl, benzimidazolyl, quinolinyl, isoquinolinyl,

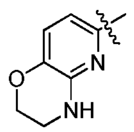, 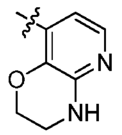, 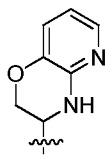, 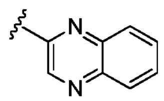, 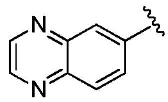, 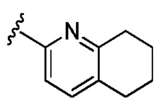, 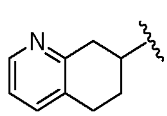, 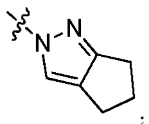;

and the like.

As used herein, the term "halo" or "halogen" radical is defined to encompass F, Cl, Br, or I.

The term "substitution" means that one or more (e.g., one, two, three, or four) hydrogens on a specified atom are replaced by a selection from the indicated radicals, provided that the normal valence of the specified atom in the present case is not exceeded and the substitution leads to a stable compound. A combination of substituents and/or variables is permissible only when such a combination leads to a stable compound.

If a substituted radical is described as "optionally...substituted," the substituted radical may be (1) unsubstituted, or (2) substituted. If a carbon of a substituted radical is described as being optionally substituted with one or more substituents in a list of substituents, one or more hydrogens (to the extent of any present hydrogens) on the carbon may be independently and/or together replaced with independently selected optional substituents. If a nitrogen of a substituted radical is described as being optionally substituted with one or more substituents in a list of substituents, one or more hydrogens (to the extent of any present hydrogens) on the nitrogen may each be replaced with independently selected optional substituents.

If a substituent is described as being "independently selected from" a group, each substituent is selected independently of the others. Therefore, each substituent may be the same as or different from another (other) substituent(s).

As used herein, the term "one or more" means 1 or more than 1, such as 2, 3, 4, 5, or 10, under reasonable conditions.

Unless otherwise specified, as used herein, the points of attachment of a substituent may be from any suitable positions at the substituent.

When a bond of a substituent is shown as a bond connecting two atoms through a ring, such a substituent may bond to any ring-forming atom in the substitutable ring.

The present disclosure further includes all pharmaceutically acceptable isotopically-labelled compounds, which are the same as the compounds of the present disclosure, except that one or more atoms are replaced with atoms which have the same atomic number, but an atomic mass or mass number different from the atomic mass or mass number

predominantly found in nature. Examples of isotopes suitable for inclusion in the compounds of the present disclosure include, but are not limited to, isotopes of hydrogen (e.g., deuterium ($^2H$), tritium ($^3H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulphur (e.g., $^{35}S$). Certain isotopically labelled compounds (e.g., those incorporating a radioisotope) of the present disclosure are useful in drug and/or substrate tissue distribution study (e.g., analysis). Radioisotopes tritium (i.e., $^3H$) and carbon-14 (i.e., $^{14}C$) are particularly preferred for their ease of incorporation and detectability. Substitutions with positron emission isotopes (e.g., $^{11}C$, $^{18}F$, $^{15}O$, and $^{13}N$) may be used to test substrate receptor occupancy in positron emission tomography (PET) studies. The isotopically-labelled compounds of the present disclosure may be prepared by methods analogous to those described in the accompanying routes and/or examples and preparations by replacing a non-isotopically labelled reagent with an appropriate isotopically labelled reagent. Pharmaceutically acceptable solvates of the present disclosure include those in which the crystallisation solvent may be substituted with an isotope, for example, $D_2O$, acetone-$d_6$, or DMSO-$d_6$.

The term "stereoisomer" means an isomer formed due to at least one asymmetric centre. In a compound with one or more (e.g., one, two, three, or four) asymmetric centres, its exo/meso mixtures, single enantiomers, and diastereomer mixtures and individual diastereomers may be produced. Specific individual molecules may also exist as geometric isomers (cis/trans). Similarly, the compound of the present disclosure may exist in a mixture of two or more structurally different forms (commonly referred to as tautomers) in rapid equilibrium. Representative examples of tautomers include keto-enol tautomers, phenol-keto tautomers, nitroso-oxime tautomers, imine-enamine tautomers, and the like. For example, nitroso-oximes may exist in equilibrium in the following tautomeric forms in solution:

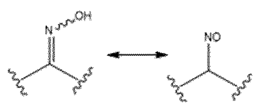.

It should be understood that the scope of the present disclosure encompasses all such isomers or mixtures thereof in any proportions (for example, 60%, 65%, 70%, 75%, 80%, 85%, 90%, 95%, 96%, 97%, 98%, 99%).

A solid line (), a solid wedge (), or a dashed wedge () may be used herein to depict chemical bonds of the compounds of the present disclosure. The use of solid lines to depict bonds to asymmetric carbon atoms is intended to indicate that all possible stereoisomers at that carbon atom (e.g., specific enantiomers or racemic mixtures) are included. The use of solid or dashed wedges to depict bonds to asymmetric carbon atoms is intended to indicate that the stereoisomers shown exist. When present in a racemic mixture, solid and dashed wedges are used to define relative stereochemistry, rather than absolute stereochemistry. Unless otherwise specified, the compound of the present disclosure is intended to exist in the form of stereoisomers (including cis and trans isomers, optical isomers (such as R and S enantiomers), diastereomers, geometric isomers, rotamers, conformational isomers, atropisomers, and mixtures thereof). The compound of the present disclosure may exhibit more than one type of isomerism and be composed of mixtures thereof (e.g., racemic mixtures and diastereomeric pairs).

The present disclosure encompasses all possible crystalline forms or polymorphs of the compound of the present disclosure, which may be a single polymorph or a mixture of more than one polymorph at any ratio.

Eutecticum refers to the fact that the active molecules of a drug and additional physiologically acceptable molecules of acids, bases, salts, and non-ionic compounds are connected by hydrogen bonds, $\pi$-$\pi$ stacking, van der Waals forces, and additional non-covalent bonds to be combined in the same crystal lattice.

It should also be understood that some compounds of the present disclosure may exist in free form for treatment, or, where appropriate, in the form of pharmaceutically acceptable derivatives thereof. In the present disclosure, pharmaceutically acceptable derivatives include, but are not limited to, pharmaceutically acceptable salts, esters, solvates, N-oxides, metabolites, or prodrugs, which, after being administered to patients in need thereof, can directly or indirectly provide the compound of the present disclosure or metabolites or residues thereof. Therefore, when the "compound of the present disclosure" is referred to herein, it is also intended to encompass the above various derivative forms of the compound.

Pharmaceutically acceptable salts of the compound of the present disclosure include both acid addition salts and base addition salts, for example, hexafluorophosphate, and meglumine salt. For a review of suitable salts, see Stahl and Wermuth, "Handbook of Pharmaceutical Salts: Properties, Selection, and Use" (Wiley-VCH, 2002).

As used herein, the term "ester" means an ester derived from the compound of each general formula in the present disclosure, which includes physiologically hydrolysable esters (hydrolysable under physiological conditions to release the compound of the present disclosure in the form of free acid or alcohol). The compound of the present disclosure itself may also be an ester itself.

The compound of the present disclosure may exist in the form of solvate (preferably hydrate), where the compound of the present disclosure comprises a polar solvent as a structural element of the crystal lattice of the compound, especially, for example, water, methanol, or ethanol. The amount of the polar solvent, especially water, may be stoichiometric or non-stoichiometric.

Those skilled in the art will understand that, since available lone pairs of electrons are required to oxidize nitrogen to form oxides, not all nitrogen-containing heterocyclic rings can form *N*-oxides. Those skilled in the art can recognize nitrogen-containing heterocyclic rings that can form *N*-oxides. Those skilled in the art can also recognize that tertiary amines can form *N*-oxides. The synthesis methods for the preparation of *N*-oxides of heterocyclic rings and tertiary amines are well known to those skilled in the art, including but not limited to the use of peroxyacids such as peroxyacetic acid and m-chloroperoxybenzoic acid (MCPBA), hydrogen peroxide, alkyl hydroperoxides such as tert-butyl hydroperoxide and sodium perborate, and dioxirane such as dimethyl dioxirane to oxidize heterocyclic rings and tertiary amines. These methods for the preparation of *N*-oxides have been widely described and summarized in literature, for example, T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The present disclosure further includes, within its scope, metabolites of the compound of the present disclosure, i.e., substances formed in vivo from the administered compound of the present disclosure. Such products may be produced by, for example, oxidation, reduction, hydrolysis, amidation, deamidation, esterification, enzymolysis, and the like of the administered compound. Therefore, the present disclosure includes metabolites of the compound of the present disclosure, including compounds prepared by exposing a mammal to the compound of the present disclosure for a period of time sufficient to produce its metabolites.

The present disclosure further includes, within its scope, the prodrugs of the compound of the present disclosure, which are certain derivatives of the compound of the present disclosure that may themselves have less pharmacological activity or no pharmacological activity, and that may be converted into the compound of the present disclosure having the desired activity by, for example, hydrolytic cleavage when administered into or onto the human body. Generally, such prodrugs are functional group derivatives of the compound, which are easily converted into the desired therapeutically active compound in vivo. Additional information on the use of prodrugs may be found in "Pro-drugs as Novel Delivery Systems", Volume 14, ACS Symposium Series (T. Higuchi and V. Stella). The prodrugs of the present disclosure may be prepared by, for example, substituting appropriate functional groups present in the compound of the present disclosure with certain moieties known to those skilled in the art as "pro-moiety (for example, as described in "Design of Prodrugs", H. Bundgaard (Elsevier, 1985))".

The present disclosure further includes the compound of the present disclosure comprising protective groups. In any process of preparing the compound of the present disclosure, protection of sensitive groups or reactive groups on any related molecules may be necessary and/or desirable, thereby forming a chemically protected form of the compound of the present disclosure. This may be achieved by conventional protective groups, for example, those protective groups as described in T. W. Greene & P. G. M. Wuts, Protective Groups in Organic Synthesis, John Wiley & Sons, 1991. These references are incorporated herein by reference. The protective groups may be removed at an appropriate subsequent stage using methods known in the art.

The term "about" means within $\pm 10\%$, preferably within $\pm 5\%$, more preferably within $\pm 2\%$, of said value.

"Pharmaceutically acceptable carriers" in the present disclosure refer to diluents, adjuvants, excipients, or vehicles which are administered together with a therapeutic agent, and are, within the scope of sound medical judgement, suitable for contact with the tissues of human beings and/or other animals without excessive toxicity, irritation, allergic reaction, or other problems or complications commensurate with a reasonable benefit/risk ratio.

Pharmaceutically acceptable carriers useful in the pharmaceutical composition of the present disclosure include, but are not limited to, sterile liquid. Examples of suitable pharmaceutically acceptable carriers are as described in Remington's Pharmaceutical Sciences (1990).

The compound of the present disclosure or a pharmaceutical composition comprising it may act systemically and/or locally. For this purpose, they may be administered through a suitable route.

For these administration routes, the compound of the present disclosure or a pharmaceutical composition comprising it may be administered in a suitable dosage form.

The term "effective amount" as used herein refers to an amount of a compound that, after being administered, will relieve one or more symptoms of the disease being treated to a certain extent.

The dosage regimen may be adjusted to provide the optimal desired response. For example, a single bolus may be administered, several divided doses may be administered over time, or the dose may be proportionally reduced or increased as indicated by the exigencies of the treatment. It should be noted that the dose may vary with the type and severity of the condition to be alleviated, and may include single or multiple doses. It should be further understood that for any particular individual, the specific dosage regimen should be adjusted over time according to the needs of the individual and the professional judgement of the person administering the composition or supervising the administration of the composition.

The dosage of the compound of the present disclosure to be administered will depend on the individual being treated, the severity of the disease or condition, the rate of administration, the disposal of the compound, and the judgement of the prescribing physician. In general, the effective dosage ranges from about 0.0001 to about 50 mg per kg body weight per day. In some cases, dosage levels not higher than the lower limit of the aforesaid range may be adequate, while in other cases still larger doses may be employed without causing any harmful side effect, provided that such larger doses are first divided into several smaller doses for administration throughout the day.

The content or amount of the compound of the present disclosure in the pharmaceutical composition may be from about 0.01 mg to about 1,000 mg.

Unless otherwise specified, the term "treating" as used herein means reversing, alleviating, inhibiting the progress of, or preventing the disorder or condition to which such term applies, or one or more symptoms of such disorder or condition.

The "individual" as used herein includes human or non-human animals. Exemplary human individuals include human individuals suffering from diseases (such as the diseases described herein) (referred to as patients) or normal individuals. In the present disclosure, "non-human animals" include all vertebrates, for example, non-mammals (such as birds, amphibians, reptiles) and mammals, for example, non-human primates, livestock, and/or domesticated animals (such as sheep, dogs, cats, cows, and pigs).

In some embodiments, the compound of the present disclosure may be administered in combination with one or more additional therapeutic agents or prophylactic agents (for example, additional drugs for treating a cancer or neoplastic disease). In some embodiments, the method of the present disclosure may also include the administration of one or more additional therapeutic agents or prophylactic agents (e.g., additional drugs for treating a cancer or a neoplastic disease).

## Pharmaceutical Use and Treatment Method

In some embodiments, the present disclosure provides the use of a compound of Formula I, a stereoisomer, tautomer, or mixture of stereoisomer and tautomer thereof, an N-oxide thereof, a pharmaceutically acceptable salt, eutecticum, polymorph, or solvate thereof, or a stable isotope derivative, metabolite, or prodrug thereof in the preparation of a drug for the treatment or adjuvant treatment of a disease or condition associated with an RET drug-resistant mutation (e.g., one or more mutations selected from the group consisting of G810R, G810S, G810C, Y806C, Y806N, and V738A) in an individual:

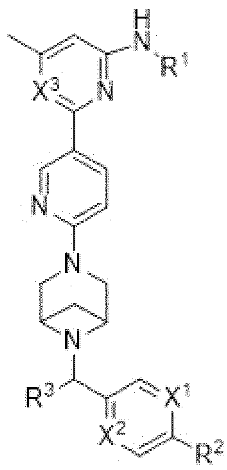

Formula I

wherein:

$R^1$ is selected from 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;
$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl,

halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;

$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and

$X^1$, $X^2$ and $X^3$ are each independently selected from CH and N.

In some preferred embodiments, in the compound of Formula I, a stereoisomer, tautomer, or mixture of stereoisomer and tautomer thereof, an N-oxide thereof, a pharmaceutically acceptable salt, eutecticum, polymorph, or solvate thereof, or a stable isotope derivative, metabolite, or prodrug thereof as related to in the present disclosure, the compound of Formula I is a compound 1:

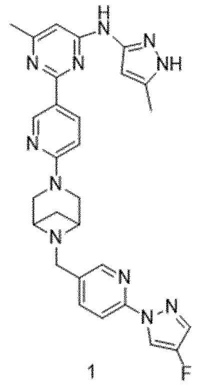

1

For example, the pharmaceutically acceptable salt of the compound is a fumarate of the compound 1.

In some embodiments, the present disclosure provides the compound of Formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof for use in the treatment or adjuvant treatment of a disease or condition associated with an RET drug-resistant mutation (e.g., one or more mutations selected from the group consisting of G810R, G810S, G810C, Y806C, Y806N, and V738A) in an individual.

In some embodiments, the present disclosure provides a method for the treatment or adjuvant treatment of a disease or condition associated with an RET drug-resistant mutation (e.g., one or more mutations selected from the group consisting of G810R, G810S, G810C, Y806C, Y806N, and V738A) in an individual, wherein the method comprises administering to the individual an effective amount of the compound of Formula I, the stereoisomer, tautomer, or mixture of stereoisomer and tautomer thereof, the N-oxide thereof, the pharmaceutically acceptable salt, eutecticum, polymorph or solvate thereof, or the stable isotope derivative, metabolite or prodrug thereof.

In some embodiments, the disease or condition associated with an RET drug-resistant mutation is a drug-resistant disease, preferably a drug-resistant cancer or tumour or irritable bowel syndrome; the caner or tumour is, for example, an advanced cancer or tumour or a metastatic cancer or tumour; the cancer or tumour is preferably lung cancer (e.g., non-small cell lung cancer), breast cancer, head and neck cancer, rectal cancer, liver cancer, lymphoma, thyroid cancer (e.g., medullary thyroid cancer or papillary thyroid cancer), colon cancer, multiple myeloma, melanoma, glioma, cerebroma or sarcoma.

In some embodiments, the drug-resistant disease is a disease resistant to Selpercatinib and/or Pralsetinib.

In some embodiments, the drug-resistant disease is non-small cell lung cancer (e.g., RET fusion-positive non-small cell lung cancer), medullary thyroid cancer (e.g., advanced or metastatic medullary thyroid cancer) or thyroid cancer (e.g., advanced or metastatic RET fusion-positive thyroid cancer) resistant to Selpercatinib and/or Pralsetinib.

In some embodiments, the present disclosure provides the use of the compound of Formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof in the preparation of a drug for the modulation (e.g., reduction or inhibition) of abnormal RET activity associated with an RET drug-resistant mutation (e.g., one or more mutations selected from the group consisting of G810R, G810S, G810C, Y806C, Y806N, and V738A).

In some embodiments, the present disclosure provides the compound of formula I, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof for the modulation (e.g., reduction or inhibition) of abnormal RET activity associated with an RET drug-resistant mutation (e.g., one or more mutations selected from the group consisting of G810R, G810S, G810C, Y806C, Y806N, and V738A).

In some embodiments, the present disclosure provides a method for the modulation (e.g., reduction or inhibition) of abnormal RET activity associated with an RET drug-resistant mutation (e.g., one or more mutations selected from the group consisting of G810R, G810S, G810C, Y806C, Y806N, and V738A), wherein the method comprises administrating an effective amount of the compound of Formula I, the stereoisomer, tautomer, or mixture of stereoisomer and tautomer thereof, the N-oxide thereof, the pharmaceutically acceptable salt, eutecticum, polymorph or solvate thereof, or the stable isotope derivative, metabolite or prodrug thereof.

In some embodiments, the RET drug-resistant mutation is one or more mutations selected from the group consisting of G810R, G810S, and G810C.

In some embodiments, the RET drug-resistant mutation is selected from one or more of RET solvent front mutation and RET hinge mutation.

In some embodiments, the drug-resistant mutation is RET solvent front mutation.

In some embodiments, the RET drug-resistant mutation is an 810 mutation, such as G810 mutation.

In a preferred embodiment, the RET drug-resistant mutation is one or more mutations selected from the group consisting of G810R, G810S, and G810C.

In some embodiments, the compound has a structure represented by Formula I-A:

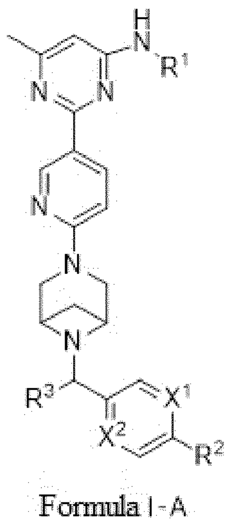

Formula I-A

wherein $R^1$, $R^2$, $R^3$, $X^1$ and $X^2$ are as defined above for Formula I.

In some embodiments, the compound has a structure represented by Formula I-B:

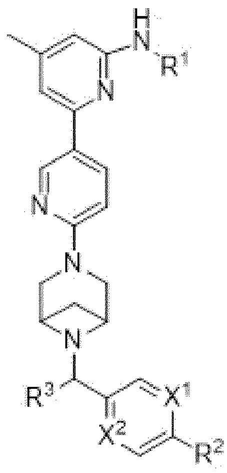

Formula I-B

wherein $R^1$, $R^2$, $R^3$, $X^1$ and $X^2$ are as defined above for Formula I.

In some embodiments, in the use of the compounds of Formula I, Formula I-A and Formula I-B provided by

the present disclosure, $R^1$ is 5-10-membered heteroaryl which is optionally substituted with one or more substituents independently selected from the group consisting of hydroxyl, halogen, CN, $C_{1-4}$ alkyl, $C_{1-4}$ haloalkyl, $C_{3-6}$ cycloalkyl and $C_{3-6}$ cycloalkoxy.

In some embodiments, in the use of the compounds of Formula I, Formula I-A and Formula I-B provided by the present disclosure, $R^1$ is 5-6-membered heteroaryl which is optionally substituted with one or more substituents independently selected from the group consisting of halogen, $C_{1-3}$ alkyl, and $C_{3-6}$ cycloalkyl.

In some embodiments, in the use of the compounds of Formula I, Formula I-A and Formula I-B provided by the present disclosure, $R^1$ is pyrazolyl which is optionally substituted with one or more substituents independently selected from the group consisting of halogen, $C_{1-3}$ alkyl (e.g., methyl) and $C_{3-6}$ cycloalkyl (e.g., cyclopropyl).

In some embodiments, in the use of the compounds of Formula I, Formula I-A and Formula I-B provided by the present disclosure, $R^1$ is pyrazolyl substituted with methyl (e.g., 5-methyl-1H-pyrazol-3-yl or 1-methyl-1H-pyrazol-4-yl) or pyrazolyl substituted with cyclopropyl (e.g., 5-cyclopropyl-1H-pyrazol-3-yl).

In some embodiments, in the use of the compounds of Formula I, Formula I-A and Formula I-B provided by the present disclosure, $R^2$ is selected from the group consisting of halogen (e.g., Cl), $C_{1-6}$ alkyl (e.g., methyl) and 5-6-membered heteroaryl, wherein the alkyl and heteroaryl are each optionally substituted with one or more substituents independently selected from the group consisting of halogen, $C_{1-4}$ alkyl, $C_{1-4}$ hydroxyalkyl, $C_{1-4}$ heteroalkyl, $C_{3-6}$ cycloalkyl and $C_{3-6}$ cycloalkoxy.

In some embodiments, in the use of the compounds of Formula I, Formula I-A and Formula I-B provided by the present disclosure, $R^2$ is 5-6-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy.

In some embodiments, in the use of the compounds of Formula I, Formula I-A and Formula I-B provided by the present disclosure, $R^2$ is 5-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy.

In some embodiments, in the use of the compounds of Formula I, Formula I-A and Formula I-B provided by the present disclosure, $R^2$ is pyrrolyl, pyrazolyl, imidazolyl, furanyl or thiazolyl, which is optionally substituted with one or more substituents independently selected from the group consisting of F, Cl, methyl,

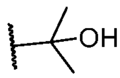,

cyclopropyl and -O-cyclopropyl.

In some embodiments, in the use of the compounds of Formula I, Formula I-A and Formula I-B provided by the present disclosure, $R^3$ is selected from H, $C_{1-6}$ alkyl and $C_{3-6}$ cycloalkyl.

In some embodiments, in the use of the compounds of Formula I, Formula I-A and Formula I-B provided by the present disclosure, $R^3$ is selected from Hand $C_{1-6}$ alkyl.

In some embodiments, in the use of the compounds of Formula I, Formula I-A and Formula I-B provided by the present disclosure, $R^3$ is H or methyl.

In some embodiments, in the use of the compounds of Formula I, Formula I-A and Formula I-B provided by the present disclosure, $X^1$ is CH or N; and preferably $X^1$ is N.

In some embodiments, in the use of the compounds of Formula I, Formula I-A and Formula I-B provided by the present disclosure, $X^2$ is CH or N; and preferably, $X^2$ is CH.

In some embodiments, in the use of the compounds of Formula I provided by the present disclosure, $X^3$ is CH or N; and preferably, $X^3$ is N.

The present disclosure encompasses any combination of the above embodiments.

In some embodiments, the compounds include, but are not limited to:

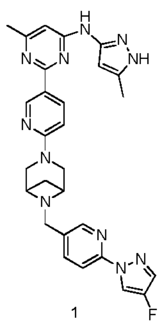
1
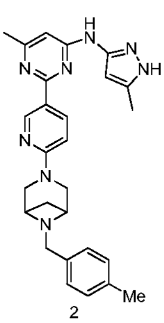
2
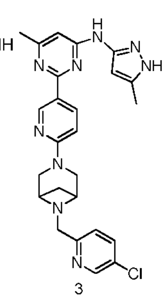
3
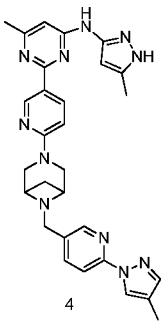
4
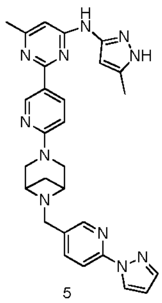
5
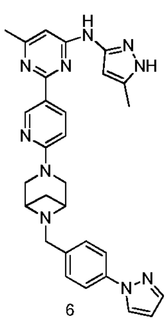
6
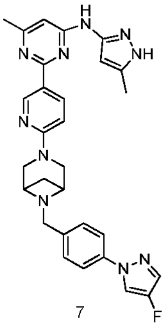
7

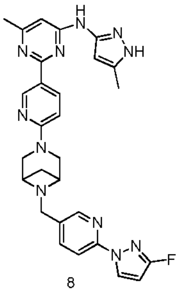
8
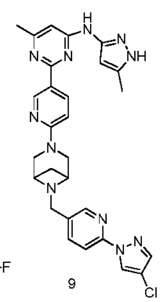
9
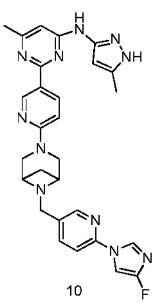
10
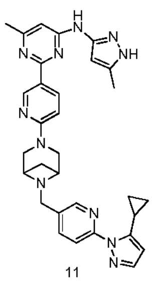
11
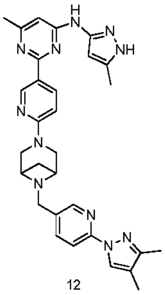
12
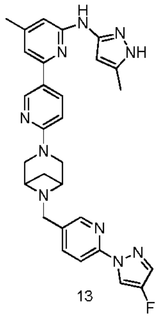
13
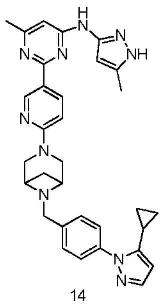
14

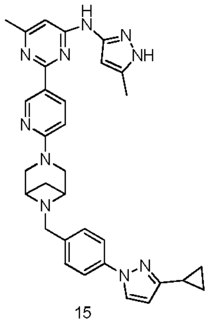
15
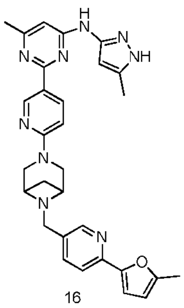
16
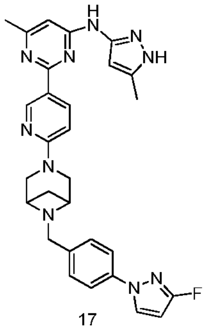
17
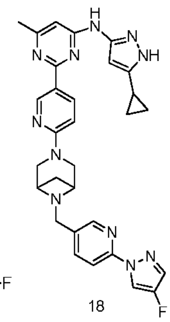
18
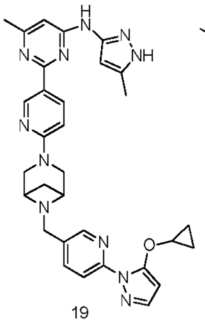
19
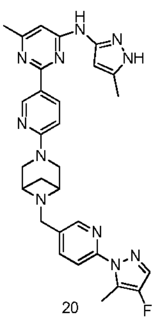
20

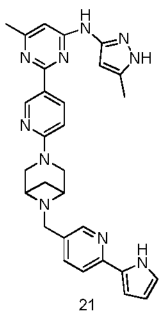
21
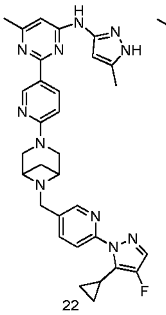
22
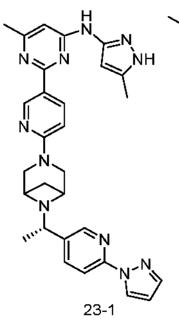
23-1
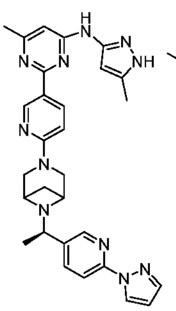
23-2
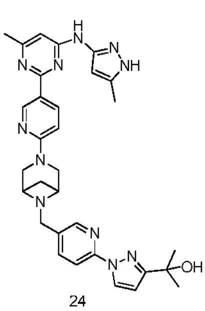
24
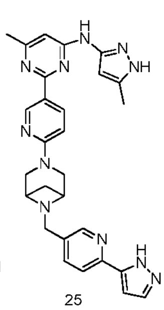
25
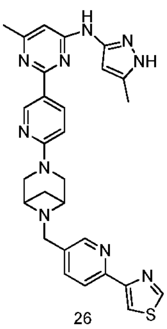
26

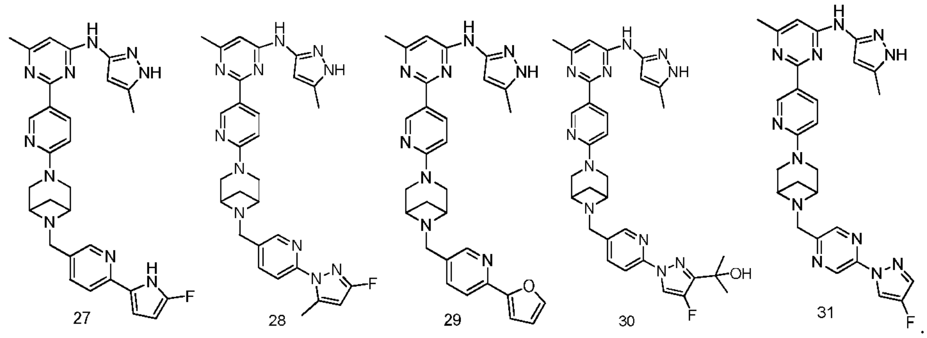

27 28 29 30 31 .

In some embodiments, the compound is:

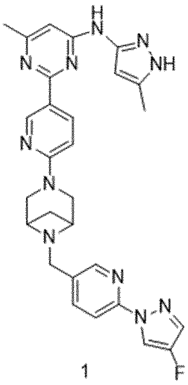

1 .

In some embodiments, the pharmaceutically acceptable salt is a fumarate.

In some embodiments, the pharmaceutically acceptable salt of the compound is:

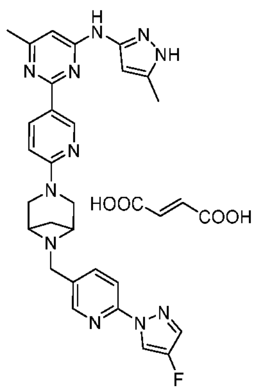

.

In some embodiments, the compound of the present disclosure may be prepared by the method described in WO 2020/168939 A1.

## Examples

The present disclosure will be further described below in combination with examples, but the provision of these examples is not intended to limit the scope of the present disclosure.

The abbreviations as used herein have the following meanings:

| Abbreviation | Meaning | h | Hour |
|---|---|---|---|
| allyl | Allyl | HPLC | High-performance liquid chromatography |
| BINAP | 2,2'-bis(diphenylphosphino)-1,1'-binaphthyl | KHMDS | Potassium bis(trimethylsilyl)amide |
| BINOL | 1,1'-binaphthol | KOAc | Potassium acetate |
| $B_2(pin)_2$ | Bis(pinacolato)diboron | LC-MS | Liquid chromatography-mass spectrometry |
| $CD_3OD$ | Deuterated methanol | LDA | Lithium diisopropylamide |
| DCE | 1,2-dichloroethane | LiHMDS | Lithium bis(trimethylsilyl)amide |
| DCM/$CH_2Cl_2$ | Dichloromethane | Me | Methyl |
| DIEA/DIPEA | N,N-diisopropylethylamine | MPLC | Medium pressure liquid chromatography |
| DMA/DMAC | Dimethylacetamide | MS | Mass spectrometry |
| DME | Dimethoxyethane | min | Minute |
| DMF | *N,N*-dimethylformamide | NaHMDS | Sodium bis(trimethylsilyl)amide |
| DMSO-$d_6$ | Deuterated dimethyl sulfoxide | NIS | N-iodosuccinimide |
| DMSO | Dimethyl sulfoxide | NMP | N-methylpyrrolidone |
| Dppf | 1,1-bis (diphenylphosphine) ferrocene | NMR | Nuclear magnetic resonance |
| EA/EtOAc | Ethyl acetate | $PCy_3$ | Tricyclohexylphosphine |
| Et | Ethyl | Pd | Palladium |
| $Pd(acac)_2$ | Bis(acetylacetone)palladium | r.t. | Room temperature |
| $Pd(dba)_2$ | Bis(dibenzylideneacetone)palladium | RuPhos | 2-dicyclohexylphosphino-2',6'-diisopropoxy-1,1'-diphenyl |
| $Pd_2(dba)_3$ | Tris(dibenzylideneacetone)dipalladium | SPhos | 2-dicyclohexylphosphino-2',6'-dimethoxy-biphenyl |
| $Pd(dppf)Cl_2$ | 1,1-bis(diphenylphosphino)ferroce | TEA | Triethylamine |
| $Pd(OAc)_2$ | ne dichloropalladium Palladium acetate | TFA | Trifluoroacetic acid |
| $Pd(PPh_3)_4$ | Tetrakis(triphenylphosphine)palladium | THF | Tetrahydrofuran |
| | | TLC | Thin-layer chromatography |
| PE | Petroleum ether | XantPhos | 4,5-bisdiphenylphosphino-9,9-dimethylxanthene |
| Prep-HPLC | Preparative high-performance liquid chromatography | XPhos | 2-dicyclohexylphosphino-2',4',6'-triisopropylbiphenyl |
| RET | Rearranged during transfection | | |

The compound of the present disclosure was separated and purified by preparative TLC, silica gel column chromatography, Prep-HPLC, and/or Flash column chromatography, and its structure was validated by $^1H$ NMR and/or MS. The reaction was monitored by TLC or LC-MS.

A Bruker superconducting nuclear magnetic resonance spectrometer (model AVACE III HD 400 MHz) was used for $^1H$ NMR spectroscopy.

Aglient 1260 Infinity/Aglient 6120 Quadrupole was used for LC/MS.

Silica gel GF 254 was used as the stationary phase for TLC.

200-300 mesh silica gel (Qingdao Marine) was generally used as the stationary phase for column chromatography.

A Biotage flash column chromatograph was used for flash column chromatography.

Agilent 1260, Waters 2489, and GeLai 3500 chromatographs were used for Prep-HPLC.

A BiotageInitiator microwave reactor was used for microwave reaction.

In the following examples, unless otherwise specified, the reaction temperature was room temperature (15-30°C).

Reagents used in the present disclosure were purchased from Acros Organics, Aldrich Chemical Company, or Terbo Chemical and the like.

**Example 1: 2-(6-(6-((6-4-chloro-1H-pyrazol-1-yl)pyridin-3-yl)methyl)-3,6-diazabicyclo[3.1.1]heptan-3-yl)pyridin-3-yl)-6-methyl-N-(5-methyl-1H-pyrazol-3-yl)pyrimidin-4-amine (Compound 9)**

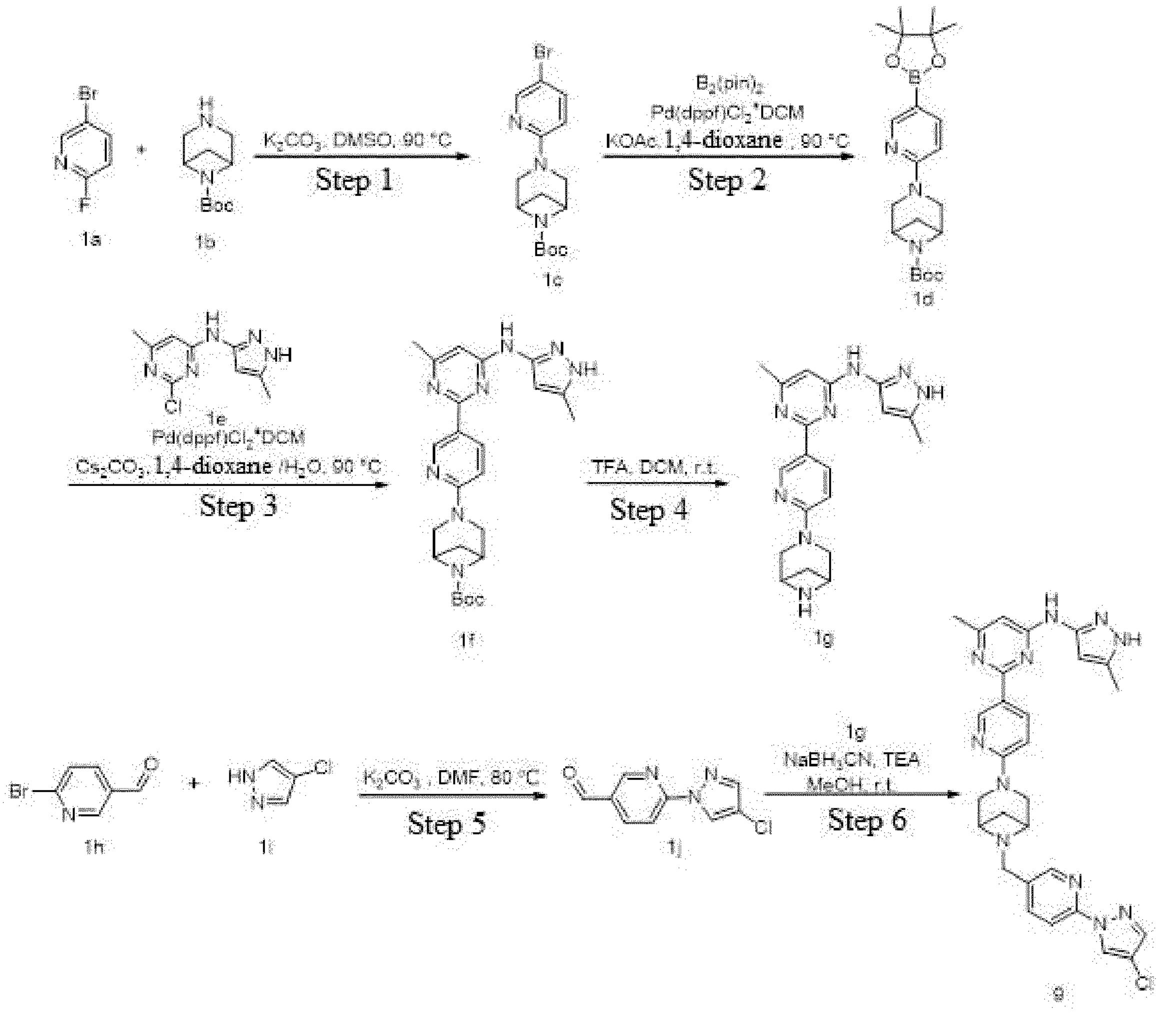


**Step 1: Tert-butyl 3-(5-bromopyridin-2-yl)-3,6-diazabicyclo [3.1.1] heptan-6-carboxylate (Compound 1c)**

Compound 1a (1.50 g) and Compound 1b (1.77 g) were successively added to a 100 mL single-necked flask, and then DMSO (20.0 mL) and $K_2CO_3$ (5.83 g) were successively added. The mixture was heated to 90°C, and stirred under the protection of nitrogen at this temperature for 20 h. After completion of the reaction, the reaction mixture was cooled to room temperature, diluted with water (100 mL), and extracted with EA. The organic phases were combined, washed with saturated brine, dried over anhydrous sodium sulphate, filtered, concentrated under reduced pressure, and separated and purified by flash silica gel column chromatography (PE:EA=5:1), to provide Compound 1c (2.03 g). MS m/z (ESI): 354.1 $[M+H]^+$.

**Step 2: Tert-butyl 3-(5-(4,4,5,5-tetramethyl-1,3,2-dioxaborolan-2-yl) pyridin-2-yl)-3,6-diazabicyclo [3.1.1] heptan-6-carboxylate (Compound 1d)**

Compound 1c (2.03 g), $B_2(pin)_2$ (4.01 g), KOAc (1.55 g), 1,4-dioxane (15.0 mL), and $Pd(dppf)Cl_2{\cdot}DCM$ (644.67 mg) were successively added into a 100 mL single-necked flask, and were heated to 90°C for reaction under the protection of nitrogen. After completion of the reaction, the reaction mixture was cooled to room temperature, diluted with water (30 mL), and extracted with EA (40 mL×3). The organic phases were combined, washed with saturated brine, dried over anhydrous sodium sulphate, filtered, concentrated to dryness under reduced pressure, and separated and purified by flash silica gel column chromatography (DCM:MeOH=15:1), to provide Compound 1d (2.11 g). MS m/z (ESI): 402.3 $[M+H]^+$.

**Step 3: Tert-butyl 3-(5-(4-methyl-6-((5-methyl-1H-pyrazol-3-yl)-amino) pyrimidin-2-yl) pyridin-2-yl)-3,6-diazabicyclo [3.1.1] heptan-6-carboxylate (Compound 1f)**

Compound 1e (950 mg) was dissolved in 1,4-dioxane (50.0 mL), Compound 1d (2.11 g), $Cs_2CO_3$ (3.15 g), and water (5.0 mL) were successively added, and then $Pd(dppf)Cl_2{\cdot}DCM$ (477.83 mg) was added. The mixture was heated to 90°C, and kept for reaction under the protection of nitrogen at this temperature for 14 h. After completion of the reaction, the reaction mixture was cooled to room temperature, diluted with water (100 mL), and extracted with EA (60 mL×3). The organic phases were combined, washed with saturated brine, dried over anhydrous sodium sulphate, filtered, and then concentrated to dryness under reduced pressure, to provide Compound 1f (587.0 mg). MS m/z (ESI): 463.3 $[M+H]^+$.

**Step 4: 2-(6-(3,6-diazabicyclo [3.1.1] heptan-3-yl) pyridin-3-yl)-6-methyl-N-(5-methyl-1H-pyrazol-3-yl) pyrimidin-4-amine (Compound 1g)**

Compound 1f (1.36 g) was dissolved in DCM (20.0 mL), TFA (20.0 mL) was then added, and the mixture was kept for reaction under the protection of nitrogen at room temperature. After completion of the reaction, the reaction mixture was concentrated to dryness under reduced pressure, and separated and purified by Prep-HPLC to provide trifluoroacetate of Compound 1g (587.0 mg). MS m/z (ESI): 363.3 $[M+H]^+$.

**Step 5: 6-(4-chloro-1H-pyrazol-1-yl) nicotinaldehyde (1j)**

Compound 1h (150 mg), Compound 1i (99 mg) and $K_2CO_3$ (334 mg) were added into DMF (4 mL), heated to 80°C to react for 14 h, and the products were detected by LC-MS. After completion of the reaction, the reaction mixture was cooled to room temperature, diluted with water, and extracted with ethyl acetate (25 mL × 3). The organic layers were combined and washed with water three times, washed with saturated brine, dried over anhydrous sodium sulphate, and filtered. The filtrate was concentrated to dryness under reduced pressure, and separated and purified by silica gel plate chromatography (DCM:MeOH=10:1) to provide the target compound 1j (23 mg). MS m/z (ESI): 208.0 $[M+H]^+$.

**Step 6: 2-(6-(6-((6-(4-chloro-1H-pyrazol-1-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 9)**

Trifluoroacetate of Compound 1g (30 mg) and Compound 1j (23 mg) were dissolved in methanol (0.5 mL), and then $Et_3N$ (8 mg) and $NaBH_3CN$ (26 mg) were successively added. The reaction mixture was kept for reaction at room temperature for 20 h, and then concentrated to dryness under reduced pressure, separated and purified by Prep-HPLC to provide Compound 9 (5 mg). MS m/z (ESI): 554.2 $[M+H]^+$.

$^1H$ NMR (400 MHz, DMSO-$d_6$) δ 11.98 (s, 1H), 9.65 (s, 1H), 9.13 (d, *J*= 2.0 Hz, 1H), 8.81 - 8.74 (m, 1H), 8.47-8.41 (m, 2H), 8.03-7.97 (m, 1H), 7.96-7.92 (m, 1H), 7.86 (d, *J*= 8.4 Hz, 1H), 6.84 (br, 1H), 6.78 (d, *J* = 8.8 Hz, 1H), 6.32 (br, 1H), 3.82 - 3.67 (m, 4H), 3.66 - 3.51(m, 4H), 2.60 - 2.53 (m, 1H), 2.33 (s, 3H), 2.26 (s, 3H), 1.60 (d, *J* = 8.4 Hz, 1H).

**Example 2**: **2-(6-(6-(4-(5-cyclopropyl-1H-pyrazol-1-yl) benzyl)-3,6-diazabicyclo [3.1.1] heptan-3-yl) pyridin-3-yl)-6-methyl-N-(5-methyl-1H-pyrazol-3-yl) pyrimidin-4-amine (Compound 14)**

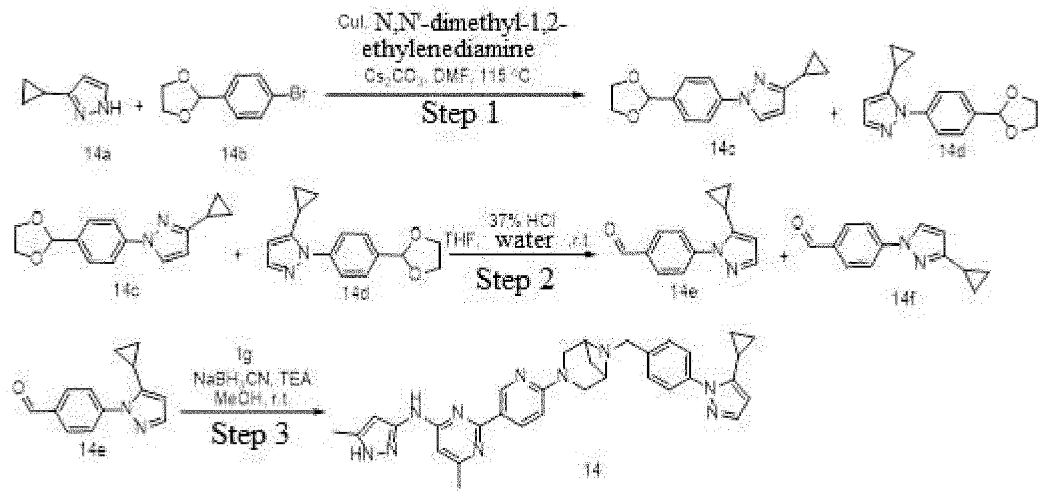


**Step 1: 1-(4-(1,3-dioxolan-2-yl) phenyl)-3-cyclopropyl-1H-pyrazole (14c) and 1-(4-(1,3-dioxolan-2-yl) phenyl)-5-cyclopropyl-1H-pyrazole (14d)**

To a round-bottom flask, 14a (472 mg), 14b (1 g), CuI (831 mg), N,N'-dimethyl-1,2-ethylenediamine (384 mg), $Cs_2CO_3$ (4.27 g) and DMF (10 mL) were successively added, heated to 115°C and kept for reaction for 3 h at this temperature under the protection of $N_2$. After the raw materials were completely converted as monitored by LC-MS, the reaction mixture was cooled to room temperature, diluted with 20 mL of water, and extracted with ethyl acetate (25 mL × 3). The organic layer was washed with water three times and dried over anhydrous sodium sulphate, and filtered. The filtrate was concentrated under reduced pressure to give a mixture of Compounds 14c and 14d (1.1 g), which was used directly without further purification for the next step of reaction. ESI-MS *(m/z):* 257.1 $[M+H]^+$.

**Step 2: 4-(5-cyclopropyl-1H-pyrazol-1-yl) benzaldehyde (14e) and 4-(3-cyclopropyl-1H-pyrazol-1-yl) benzaldehyde (14f)**

The mixture of 14c and 14d from the previous step (930 mg) was dissolved in a mixed solvent of THF (20 mL) and water (20 mL), and then a 37% HCl solution (15 mL) was added dropwise. The mixture was kept for reaction at room temperature for 18 h, and the raw materials were completely converted as monitored by LC-MS. Part of the solvent was evaporated under reduced pressure, and the reaction mixture was adjusted to pH of about 9 with saturated $NaHCO_3$, and extracted with ethyl acetate (20 mL × 3). The organic layers were combined and dried over anhydrous sodium sulphate, and filtered. The filtrate was concentrated under reduced pressure, separated and purified by silica gel plate chromatography (DCM:MeOH=10:1) to give Compound 14e (less polar compound in TLC) (400 mg) and Compound 14f (more polar compound in TLC) (20 mg). ESI-MS (*m*/*z*): 213.2 $[M+H]^+$.

**Step 3: 2-(6-(6-(4-(3-cyclopropyl-1H-pyrazol-1-yl) benzyl)-3,6-diazabicyclo [3.1.1] heptan-3-yl) pyridin-3-yl)-6-methyl-N-(5-methyl-1H-pyrazol-3-yl) pyrimidin-4-amine (Compound 14)**

Trifluoroacetate of Compound 1g (30 mg) and Compound 14e (26 mg) were dissolved in methanol (0.5 mL), and then $Et_3N$ (8 mg) and $NaBH_3CN$ (26 mg) were successively added. Thereafter, the reaction mixture was kept for reaction at room temperature for 16 h. After completion of the reaction, the reaction mixture was concentrated to dryness under reduced pressure, and separated and purified by Prep-HPLC to provide Compound 14 (22 mg). MS m/z (ESI): 559.3 $[M+H]^+$.

$^1H$ NMR (400 MHz, DMSO-$d_6$) δ 11.97 (s, 1H), 9.65 (s, 1H), 9.12 (d, *J* = 2.4 Hz, 1H), 8.43 (dd, *J* = 9.2, 2.4 Hz, 1H), 8.28 (d, *J* = 2.4 Hz, 1H), 7.68 (d, *J* = 8.4 Hz, 2H), 7.41 (d, *J* = 8.4 Hz, 2H), 6.84 (br, 1H), 6.78 (d, *J* = 8.8Hz, 1H), 6.30 (br, 1H), 6.21 (d, *J* = 2.4Hz, 1H), 3.82 - 3.65 (m, 4H), 3.64 - 3.47(m, 4H), 2.59 - 2.53 (m, 1H), 2.33 (s, 3H), 2.26 (s, 3H), 2.00 - 1.92 (m, 1H), 1.59 (d, *J*= 8.4 Hz, 1H), 0.95 - 0.88 (m, 2H), 0.75 - 0.69 (m, 2H).

**Example 3: 2-(6-(6-(4-(3-cyclopropyl-1H-pyrazol-1-yl) benzyl)-3,6-diazabicyclo [3.1.1] heptan-3-yl) pyridin-3-yl)-6-methyl-N-(5-methyl-1H-pyrazol-3-yl) pyrimidin-4-amine (Compound 15)**

Trifluoroacetate of Compound 1g (30 mg) and Compound 14f (20 mg) were dissolved in methanol (0.5 mL), and then $Et_3N$ (8 mg) and $NaBH_3CN$ (26 mg) were successively added. Thereafter, the reaction mixture was kept for

reaction at room temperature for 16 h. After completion of the reaction, the reaction mixture was concentrated to dryness under reduced pressure, and separated and purified by Prep-HPLC to provide Compound 15 (12 mg). MS m/z (ESI): 559.3 $[M+H]^+$.

$^1H$ NMR (400 MHz, DMSO-$d_6$) δ 11.79 (br, 1H), 9.66 (s, 1H), 9.12 (d, *J* = 2.4 Hz, 1H), 8.44 (dd, *J* = 8.8, 2.4 Hz, 1H), 7.59-7.45 (m, 5H), 6.83(br, 1H), 6.78 (d, *J* = 9.2Hz, 1H), 6.28 (br, 1H), 6.09 (d, *J* = 1.6 Hz, 1H), 3.85-3.72 (m, 4H), 3.66 - 3.53 (m, 4H), 2.62-2.52 (m, 1H), 2.33 (s, 3H), 2.25 (s, 3H), 1.88-1.77 (m, 1H), 1.60 (d, *J* = 8.4 Hz, 1H), 0.98 - 0.92 (m, 2H), 0.75 - 0.69 (m, 2H).

**Example 4: Fumarate of 2-(6-(6-((6-(4-fluoro-1H-pyrazol-1-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 1-A)**

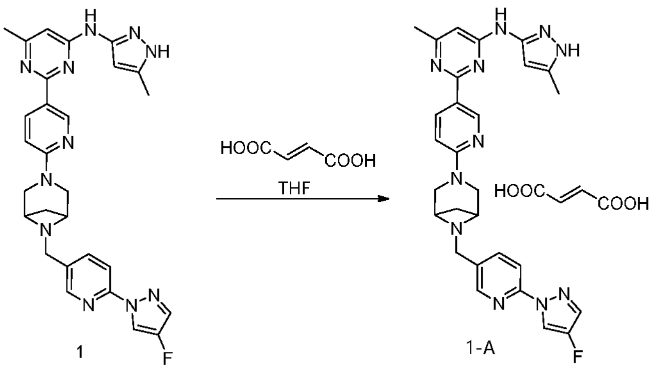

1 1-A

Compound 1 (5 g) was added to 200 mL of THF and stirred until it was completely dissolved. Then 1.56 g of fumaric acid was added slowly, and the reaction mixture was kept for reaction at 20-45°C for 46 h. After completion of the reaction, the mixture was filtered and washed with THF to obtain 7.06 g of a wet solid, which was then dried under vacuum at 40-45°C to give 4.78 g of solid powder.

$^1H$ NMR (400 MHz, DMSO-$d_6$) δ 12.43 (br, 3H), 9.66 (s, 1H), 9.13 (d, *J* = 2.4 Hz, 1H), 8.60 (d, *J* = 4.4 Hz, 1H), 8.44 (dd, *J* = 8.8, 2.4 Hz, 1H), 8.41 (d, *J* = 1.2 Hz, 1H), 7.97 (dd, *J* = 8.4, 2.0 Hz, 1H), 7.91 (d, *J* = 4.4 Hz, 1H), 7.86(d, *J* = 8.4 Hz, 1H), 6.81 (br, 1H), 6.76 (d, *J*= 8.8 Hz, 1H), 6.62(s, 2H), 6.31 (br, 1H), 3.79-3.76 (m, 4H), 3.68-3.58 (m, 4H), 2.62-2.56 (m, 1H), 2.33 (s, 3H), 2.25 (s, 3H), 1.61 (d, *J* = 8.4 Hz, 1H).

**Example 5: 2-(1-(5-((3-(5-(4-methyl-6-((5-methyl-1H-pyrazol-3-yl) amino) pyrimidin-2-yl) pyridin-2-yl)-3,6-diazabicyclo [3.1.1] heptan-6-yl) methyl) pyridin-2-yl)-1H-pyrazol-3-yl) propan-2-ol (Compound 24)**

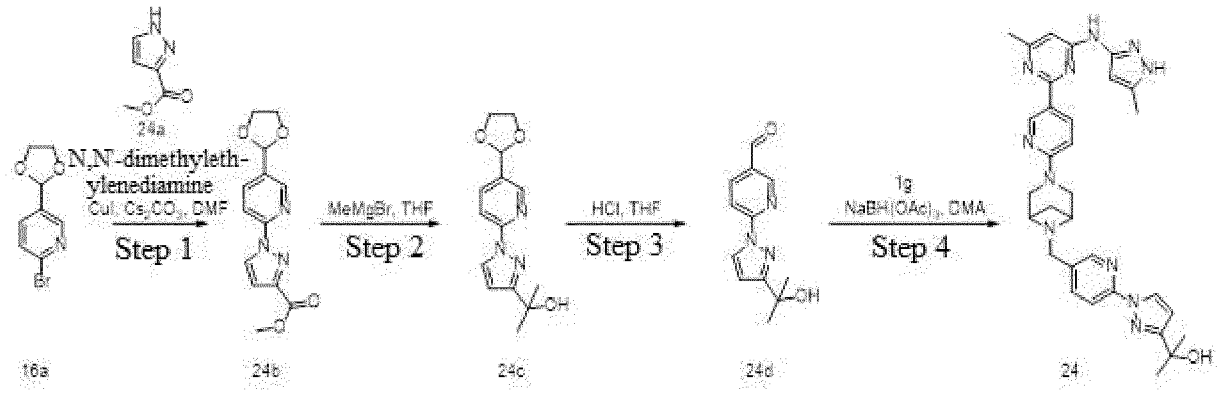

16a 24b 24c 24d 24

**Step 1: Methyl 1-(5-(1,3-dioxolan-2-yl) pyridin-2-yl)-1H-pyrazol-3-carboxylate (Compound 24b)**

Compound 16a (2.53 g) and Compound 24a (1.52 g) were dissolved in DMF (10 mL), and then N,N'-dimethylethylenediamine (379.29 mg), $Cs_2CO_3$ (7.01 g) and CuI (409.74 mg) were successively added. The reaction mixture

was heated to 90°C and kept for reaction at this temperature under the protection of nitrogen for 2 h. After completion of the reaction, the reaction mixture was cooled to room temperature, quenched with water (60 mL) and extracted with EA (30 mL × 3). The organic phases were combined, washed with saturated saline, dried over anhydrous sodium sulphate, filtered, and concentrated under reduced pressure. The filtrate was separated and purified by silica gel column chromatography (PE:EA = 50:1 to 5:1) to obtain Compound 24b (1.59 g). MS m/z (ESI): 276.0 $[M+H]^+$.

**Step 2: 2-(1-(5-(1,3-dioxolan-2-yl) pyridin-2-yl)-1H-pyrazol-3-yl) propan-2-ol (Compound 24c)**

Compound 24b (200 mg) was added to dried THF (10 mL) and allowed to cool in a dry ice ethanol bath for 15 min. Then, a solution of MeMgBr in $Et_2O$ (3 N, 0.65 mL) was slowly added dropwise. The mixture was allowed to react at this temperature for 15 min, and then warmed to room temperature to further react for 4 h. After completion of the reaction, the reaction mixture was quenched with a saturated aqueous solution of ammonium chloride (1 mL), diluted with water (30 mL), and extracted with EA (30 mL × 3). The organic phases were combined, washed with saturated saline, dried over anhydrous sodium sulphate, filtered, and concentrated to give Compound 24c (200 mg). MS m/z (ESI): 276.1 $[M+H]^+$.

**Step 3: 6-(3-(2-hydroxyprop-2-yl)-1H-pyrazol-1-yl) nicotinaldehyde (Compound 24d)**

Compound 24c (200 mg) was added to THF (5 mL), then hydrochloric acid (2 N, 4.5 mL) was added, and the reaction mixture was kept for reaction at 25°C for 12 h. After completion of the reaction, a saturated aqueous solution of $NaHCO_3$ was added to the reaction mixture to adjust pH to 7-8 and the mixture was extracted with EA (30 mL × 3). The organic phases were combined and washed with saturated saline, dried over anhydrous sodium sulphate, and filtered. The filtrate was concentrated under reduced pressure to afford Compound 24d (165 mg). MS m/z (ESI): 232.1 $[M+H]^+$.

**Step 4: Preparation of 2-(1-(5-((3-(5-(4-methyl-6-((5-methyl-1H-pyrazol-3-yl) amino) pyrimidin-2-yl) pyridin-2-yl)-3,6-diazabicyclo [3.1.1] heptan-6-yl) methyl) pyridin-2-yl)-1H-pyrazol-3-yl) propan-2-ol (Compound 24)**

Trifluoroacetate of Compound 1g was purified by Flash column chromatography under basic conditions (purification conditions: C18 reversed-phase column; mobile phase: 0.5% aqueous solution of ammonium bicarbonate, and $CH_3CN$; gradient: 0% $CH_3CN$ for elution for 5 min, 0%-100% $CH_3CN$ for gradient elution for 25 min, and 45%-65% $CH_3CN$ for product collection), concentrated, and lyophilized to obtain free amine 1g.

Compound 24d (50.5 mg) and Compound 1g (30 mg) were then added to DMA (3 mL) and stirred under the protection of nitrogen at room temperature for 1 h. $NaBH(OAc)_3$ (101 mg) was then added and the reaction was held at room temperature overnight. After completion of the reaction, water (60 mL) was added to the reaction mixture to quench the reaction and the mixture was extracted with EA (30 mL × 3). The organic phases were combined and washed with saturated saline, dried over anhydrous sodium sulphate, and filtered. The filtrate was concentrated under reduced pressure, and separated and purified by Prep-HPLC to afford Compound 24 (12 mg). MS m/z (ESI): 578.3 $[M+H]^+$.

$^1H$ NMR (400 MHz, DMSO-$d_6$) $\delta$ 11.97 (br, 1H), 9.67 (s, 1H), 9.12 (d, *J* = 2.0 Hz, 1H), 8.48-8.41 (m, 2H), 8.37 (d, *J* = 1.6 Hz, 1H), 7.94 (dd, *J* = 8.4, 2.4 Hz, 1H), 7.82 (d, *J* = 8.4 Hz, 1H), 6.82 (br, 1H), 6.79 (d, *J* = 8.8 Hz, 1H), 6.52 (d, *J* = 2.8 Hz, 1H), 6.31 (br, 1H), 5.10 (s, 1H), 3.82-3.70 (m, 4H), 3.68-3.55 (m, 4H), 2.61-2.54 (m, 1H), 2.33 (s, 3H), 2.26 (s, 3H), 1.60 (d, *J*= 8.4 Hz, 1H), 1.49 (s, 6H).

**Example 6: 2-(6-(6-((6-(3-fluoro-5-methyl-1H-pyrazol-1-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 28)**

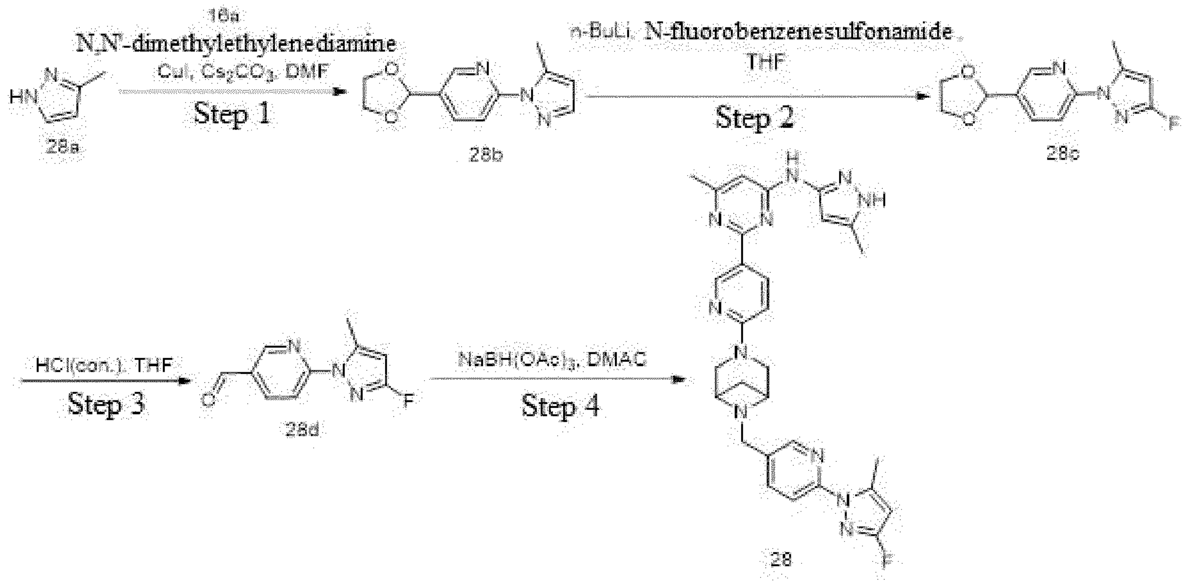


**Step 1: 5-(1,3-dioxolan-2-yl)-2-(5-methyl-1H-pyrazol-1-yl) pyridine (Compound 28b)**

Compound 28a (785 mg), Compound 16a (2000 mg), N,N'-dimethylethylenediamine (766 mg), CuI (1660 mg), and $Cs_2CO_3$ (8500 mg) were added to DMF (50 mL), and heated to 110°C to react for 12 h under the protection of nitrogen. After completion of the reaction, the reaction mixture was cooled to room temperature, quenched with water and extracted with EA. The organic phases were combined, washed with water, dried over anhydrous sodium sulphate, and filtered. The filtrate was concentrated under reduced pressure, and separated and purified by silica gel column chromatography (PE:EA=7:3) to afford Compound 28b (2010 mg). MS m/z (ESI): 232.1 $[M+H]^+$.

**Step 2: 5-(1,3-dioxolan-2-yl)-2-(3-fluoro-5-methyl-1H-pyrazol-1-yl) pyridine (Compound 28c)**

Compound 28b (600 mg) was added to anhydrous THF (30 mL), cooled to -70°C, and 1.6 mL of a 2.5 M solution of n-butyl lithium in THF was added dropwise under the protection of nitrogen, and the reaction was allowed to continue at -70°C for 1 h. Then, a solution of N-fluorobenzenesulfonamide in THF (30 mL) was added dropwise, and thereafter the reaction was allowed to continue at -70°C for 12 h. After completion of the reaction, water was added to the reaction mixture to quench the reaction, and the mixture was extracted with EA. The organic phases were combined, washed with water, dried over anhydrous sodium sulphate, and filtered. The filtrate was concentrated under reduced pressure, and separated and purified by silica gel column chromatography (PE:EA=17:3) to obtain Compound 28c (80 mg). MS m/z (ESI): 250.1 $[M+H]^+$.

**Step 3: 6-(3-fluoro-5-methyl-1H-pyrazol-1-yl) nicotinaldehyde (Compound 28d)**

Concentrated hydrochloric acid (12 N, 2 mL) was added dropwise to a solution of Compound 28c (80 mg) in a mixed solvent of THF (2 mL) and water (2 mL) to react at room temperature for 8 h. Then, the reaction mixture was adjusted to pH of about 10 with a saturated solution of $K_2CO_3$, and then extracted with EA. The organic phases were combined, dried over anhydrous sodium sulphate, and filtered. The filtrate was concentrated under reduced pressure, and separated and purified by silica gel column chromatography (DCM:MeOH=9:1) to afford Compound 28d (55 mg). MS m/z (ESI): 206.1 $[M+H]^+$.

**Step 4: 2-(6-(6-((6-(3-fluoro-5-methyl-1H-pyrazol-1-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 28)**

Compound 1g (30 mg) and Compound 28d (20 mg) were dissolved in DMAC (1 mL), allowed to react at room temperature for 1 h, then $NaBH(OAc)_3$ (70 mg) was added, and the reaction was allowed to continue at room temperature for 12 h. After completion of the reaction, the reaction mixture was diluted with ethyl acetate. The organic phase was washed with an aqueous solution of $K_2CO_3$, dried over anhydrous sodium sulphate, and filtered. The filtrate was concentrated under reduced pressure, and separated and purified by Flash column chromatography to obtain Compound 28 (8 mg). MS m/z (ESI): 552.3 $[M+H]^+$.

$^1H$ NMR (400 MHz, DMSO-$d_6$) δ 11.98 (s, 1H), 9.66 (s, 1H), 9.12 (d, *J* = 2.4 Hz, 1H), 8.45-8.42 (m, 2H), 7.97 (dd, *J* = 8.4, 2.4 Hz, 1H), 7.66 (d, *J* = 8.4 Hz, 1H), 6.84 (br, 1H), 6.78 (d, *J* = 9.2 Hz, 1H), 6.32 (br, 1H), 6.08 (d, *J* = 5.2 Hz, 1H), 3.78-3.70 (m, 4H), 3.65-3.56 (m, 4H), 2.60-2.54 (m, 1H), 2.33 (s, 3H), 2.25 (s, 3H), 2.21 (s, 3H), 1.59 (d, *J* = 8.4 Hz, 1H).

**Example 7: 2-(6-(6-((6-(furan-2-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 29)**

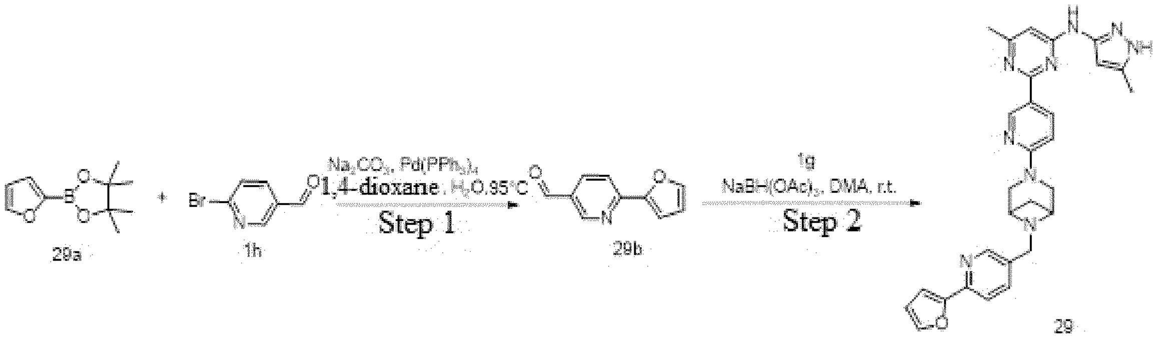


**Step 1: 6-(furan-2-yl) nicotinaldehyde (29b)**

To a round-bottom flask, 29a (312 mg), 1h (200 mg), $Na_2CO_3$ (341 mg,), $Pd(PPh_3)_4$ (62 mg), 1,4-dioxane (10 mL), and water (2.5 mL) were successively added, and heated to 95°C under the protection of nitrogen to react for 2 h. After completion of the reaction, the reaction mixture was cooled to room temperature, and separated and purified by Flash silica gel column chromatography (PE:EA=87%:13%) to give Compound 29b (150 mg). MS m/z (ESI): 174.2 $[M+H]^+$.

**Step 2: Preparation of 2-(6-(6-((6-(furan-2-yl) pyridin-3-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 29)**

Compound 1g (30 mg) and Compound 29b (21 mg) were added to DMA (1 mL) to react at room temperature for 2 h. Then, $NaBH(OAc)_3$ (70 mg) wa s added, and the reaction was allowed to continue at room temperature for 16 h. After completion of the reaction, the reaction mixture was separated and purified directly by Prep-HPLC to afford Compound 29 (30 mg). MS m/z (ESI): 520.3 $[M+H]^+$.

$^1H$ NMR (400 MHz, DMSO-$d_6$) δ 11.98 (s, 1H), 9.66 (s, 1H), 9.12 (d, *J* = 2.0 Hz, 1H), 8.52 (d, *J* = 1.6 Hz, 1H), 8.44 (dd, *J* = 9.2, 2.4 Hz, 1H), 7.87 - 7.78 (m, 2H), 7.67 (d, *J* = 8.0 Hz, 1H), 7.07 (dd, *J* = 3.2, 0.4 Hz, 1H), 6.83 (br, 1H), 6.78 (d, *J* = 9.2 Hz, 1H), 6.64 (dd, *J* = 3.2, 2.0 Hz, 1H), 6.31 (br, 1H), 3.82 - 3.69 (m, 4H), 3.65-3.47 (m, 4H), 2.59 - 2.53 (m, 1H), 2.33 (s, 3H), 2.26 (s, 3H), 1.59 (d, *J* = 8.4 Hz, 1H).

**Example 8: Fumarate of 2-(4-fluoro-1-(5-((3-(5-(4-methyl-6-((5-methyl-1H-pyrazol-3-yl) amino) pyrimidin-2-yl) pyridin-2-yl)-3,6-diazabicyclo [3.1.1] heptan-6-yl) methyl) pyridin-2-yl)-1H-pyrazol-3-yl) propan-2-ol (Compound 30-A)**

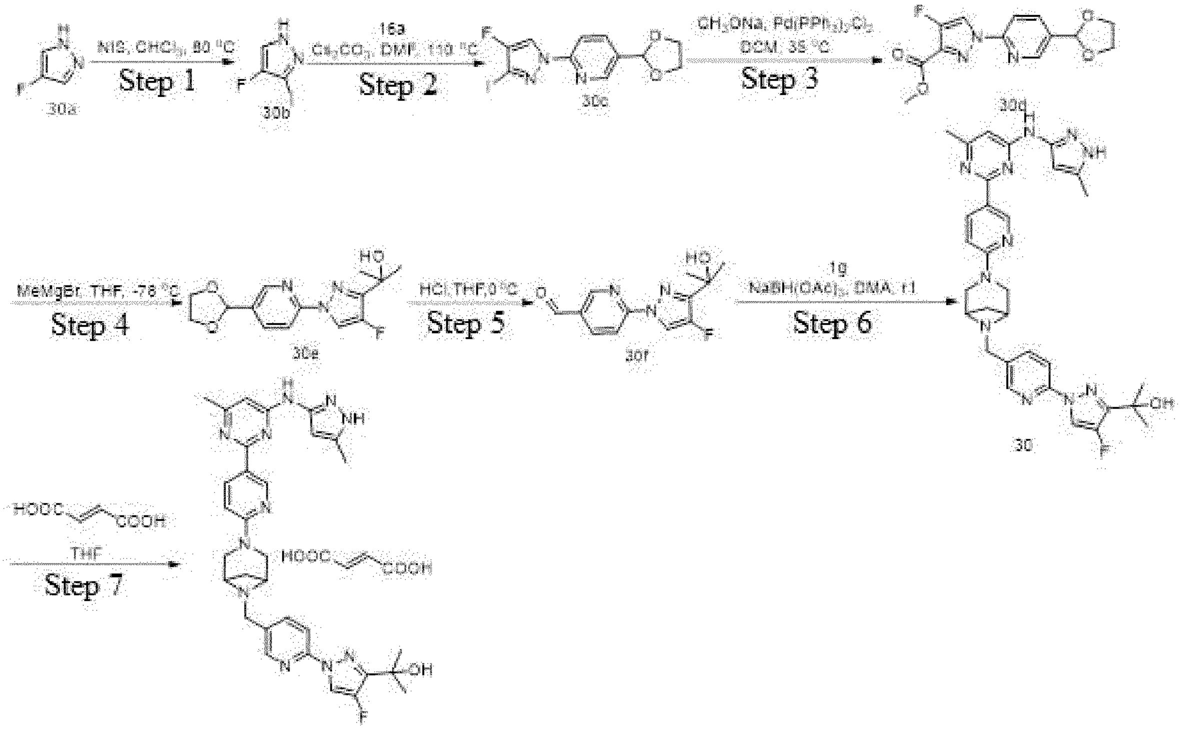


30-A

**Step 1: 4-fluoro-3-iodo-1H-pyrazole (30b)**

Compound 30a (3.0 g) was dissolved in chloroform (50 mL), and then NIS (8.36 g) was added. The mixture was heated to 80°C and allowed to react overnight under the protection of nitrogen. After completion of the reaction, the reaction mixture was cooled to room temperature, water (60 mL) was added to quench the reaction, and the reaction mixture was extracted with EA (30 mL × 3). The organic phases were combined, washed with saturated saline, dried over anhydrous sodium sulphate, filtered, concentrated, and separated and purified by silica gel column chromatography (PE:EA=7:1) to afford Compound 30b (720 mg). MS m/z (ESI): 213.0 $[M+H]^+$.

**Step 2: 5-(1,3-dioxolan-2-yl)-2-(4-fluoro-3-iodo-1H-pyrazol-1-yl) pyridine (30c)**

Compound 16a (705 mg) and Compound 30b (500 mg) were dissolved in DMF (10 mL), and then $Cs_2CO_3$ (1.75 g) was added. The mixture was heated to 110°C and allowed to react for 16 h under the protection of nitrogen. After completion of the reaction, the reaction mixture was cooled to room temperature, water (60 mL) was added to quench the reaction, and the reaction mixture was extracted with EA (30 mL × 3). The organic phases were combined, washed with saturated saline, dried over anhydrous sodium sulphate, filtered, concentrated, and separated and purified by silica gel column chromatography (PE:EA=9:1) to afford Compound 30c (720 mg). MS m/z (ESI): 361.9 $[M+H]^+$.

**Step 3: Methyl 1-(5-(1,3-dioxolan-2-yl) pyridin-2-yl)-4-fluoro-1H-pyrazol-3-carboxylate (30d)**

Compound 30c (250 mg) was dissolved in DCM (15 mL), and then MeONa (160 mg), methyl formate (721 mg), and $Pd(PPh_3)_2Cl_2$ (83 mg) were successively added. The mixture was heated to 35°C and allowed to react for 2 h under the protection of nitrogen. After completion of the reaction, the reaction mixture was cooled to room temperature, directly concentrated, and separated and purified by silica gel column chromatography (PE:EA=5:1) to obtain Compound 30d (150 mg). MS m/z (ESI): 294.1 $[M+H]^+$.

**Step 4: 2-(1-(5-(1,3-dioxolan-2-yl) pyridin-2-yl)-4-fluoro-1H-pyrazol-3-yl) propan-2-ol (30e)**

Compound 30d (180 mg) was dissolved in dried THF (15 mL), cooled in a dry ice ethanol bath at -78°C for 15 min, and then a solution of MeMgBr in ether (3 N, 0.95 mL) was slowly added dropwise. Thereafter, the reaction was kept for 15 min, and then allowed to warm to room temperature to react for 4 h. After completion of the reaction, a

saturated aqueous solution of ammonium chloride (1 mL) was added to quench the reaction, the reaction mixture was diluted with water (30 mL), and extracted with EA (30 mL × 3). The organic phases were combined, washed with saturated saline, dried over anhydrous sodium sulphate, and filtered. The filtrate was concentrated under reduced pressure to give crude Compound 30e (145 mg), which was used directly in the next step of reaction without further purification. MS m/z (ESI): 294.2 $[M+H]^+$.

**Step 5: 6-(4-fluoro-3-(2-hydroxypropan-2-yl)-1H-pyrazol-1-yl) nicotinaldehyde (30f)**

Compound 30e (300 mg) was dissolved in THF (5 mL), and then hydrochloric acid (2 N, 15 mL) was added. The mixture was allowed to react in an ice bath for 2 h. Then, to the reaction mixture, a saturated aqueous solution of $NaHCO_3$ was added to adjust pH to 7-8, and the mixture was extracted with EA (30 mL × 3). The organic phases were combined, washed with saturated saline, dried over anhydrous sodium sulphate, and filtered. The filtrate was concentrated under reduced pressure, and separated and purified by silica gel column chromatography (PE:EA=3:1) to afford Compound 30f (200 mg). MS m/z (ESI): 250.1 $[M+H]^+$.

**Step 6: 2-(4-fluoro-1-(5-((3-(5-(4-methyl-6-((5-methyl-1H-pyrazol-3-yl) amino) pyrimidin-2-yl) pyridin-2-yl)-3,6-diazabicyclo [3.1.1] heptan-6-yl) methyl) pyridin-2-yl)-1H-pyrazol-3-yl) propan-2-ol (Compound 30)**

Compound 1g (46 mg) and Compound 30f (45 mg) were dissolved in DMA (1 mL) and allowed to react at room temperature for 1 h under the protection of nitrogen. Then, $NaBH(OAc)_3$ (101 mg) was added, and the mixture was allowed to react overnight at room temperature. After completion of the reaction, water (60 mL) was added to quench the reaction, and the reaction mixture was extracted with EA (30 mL × 3). The organic phases were combined, washed with saturated saline, dried over anhydrous sodium sulphate, and filtered. The filtrate was concentrated under reduced pressure, and separated and purified by Prep-HPLC to give Compound 30 (10 mg). MS m/z (ESI): 596.3 $[M+H]^+$.

**Step 7: Fumarate of 2-(4-fluoro-1-(5-((3-(5-(4-methyl-6-((5-methyl-1H-pyrazol-3-yl) amino) pyrimidin-2-yl) pyridin-2-yl)-3,6-diazabicyclo [3.1.1] heptan-6-yl) methyl) pyridin-2-yl)-1H-pyrazol-3-yl) propan-2-ol (Compound 30-A)**

Compound 30 (117 mg, 0.19 mmol) was dissolved in THF (4.0 mL), and then fumaric acid (46 mg, 0.39 mmol) was added. The mixture was allowed to react with stirring overnight at room temperature under the protection of nitrogen. After completion of the reaction, the mixture was filtered, washed with THF, and dried under vacuum to obtain 115 mg of the fumarate.

$^1H$ NMR (400 MHz, DMSO-$d_6$) δ 12.73 (br, 2H), 9.66 (br, 1H), 9.12 (d, $J$= 2.4 Hz, 1H), 8.56 (d, $J$ = 4.8 Hz, 1H), 8.44 (dd, $J$ = 8.8, 2.4 Hz, 1H), 8.37 (d, $J$ = 2.0 Hz, 1H), 7.96 (dd, $J$ = 8.4, 2.4 Hz, 1H), 7.81 (d, $J$ = 8.4 Hz, 1H), 6.83 (br, 1H), 6.78 (d, $J$ = 8.8 Hz, 1H), 6.62 (s, 2H), 6.30 (br, 1H), 5.24 (s, 1H), 3.79-3.72 (m, 4H), 3.64-3.58 (m, 4H), 2.59 - 2.54 (m, 1H), 2.33 (s, 3H), 2.26 (s, 3H), 1.60 (d, $J$ = 8.4 Hz, 1H), 1.54 (s, 6H).

**Example 9: 2-(6-(6-((5-(4-fluoro-1H-pyrazol-1-yl) pyrazin-2-yl) methyl)-3,6-diazabicyclo [3.1.1] hept-3-yl) pyridin-3-yl)-6-methyl-N-(5-methyl-1H-pyrazol-3-yl) pyrimidin-4-amine (Compound 31)**

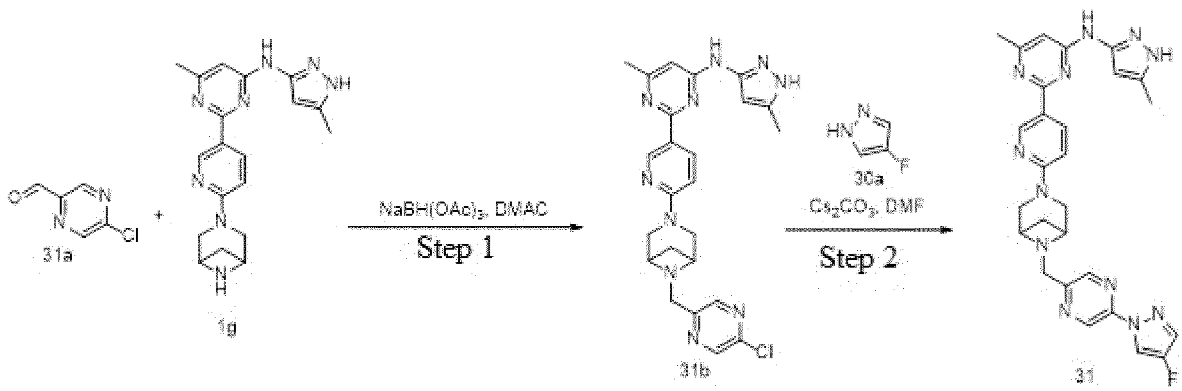

**Step 1: 2-(6-(6-((5-chloropyrazin-2-yl) methyl)-3,6-diazabicyclo [3.1.1] heptan-3-yl) pyridin-3-yl)-6-methyl-N-(5-methyl-1H-pyrazol-3-yl) pyrimidin-4-amine (31b)**

Compound 1g (150 mg) and Compound 31a (71 mg) were dissolved in DMAC (5 mL) to react at room temperature for 1 h, then $NaBH(OAc)_3$ (351 mg) was added, and the reaction was allowed to continue at room temperature for 12 h. After completion of the reaction, the reaction mixture was diluted with ethyl acetate. The organic phase was washed with an aqueous solution of $K_2CO_3$, dried over anhydrous sodium sulphate, and filtered. The filtrate was concentrated under reduced pressure, and separated and purified by Prep-TLC (DCM:MeOH=15:1) to obtain Compound 31b (110 mg). MS m/z (ESI): 489.2 $[M+H]^+$.

**Step 2: 2-(6-(6-((5-(4-fluoro-1H-pyrazol-1-yl) pyrazin-2-yl) methyl)-3,6-diazabicyclo [3.1.1] heptan-3-yl) pyridin-3-yl)-6-methyl-N-(5-methyl-1H-pyrazol-3-yl) pyrimidin-4-amine (Compound 31)**

Compound 31b (30 mg) and Compound 30a (106 mg) were dissolved in DMF (1.5 mL), $Cs_2CO_3$ (60 mg) was added, and the mixture was heated to 90°C to react with stirring for 1 h. After completion of the reaction, the reaction mixture was cooled to room temperature, diluted with ethyl acetate, and washed with water. The organic phase was dried over anhydrous sodium sulphate, and filtered. The filtrate was concentrated under reduced pressure, and separated and purified by Prep-HPLC to obtain Compound 31 (30 mg). MS m/z (ESI): 539.3 $[M+H]^+$.

$^1H$ NMR (400 MHz, DMSO-$d_6$) $\delta$ 11.99 (s, 1H), 9.66 (s, 1H), 9.12 (d, *J* = 2.4 Hz, 1H), 9.09 (d, *J* = 1.6 Hz, 1H), 8.72 (dd, *J* = 4.8, 0.8 Hz, 1H), 8.56 (d, *J* = 1.2 Hz, 1H), 8.44 (dd, *J* = 8.8, 2.0 Hz, 1H), 8.03 (d, *J* = 4.0 Hz, 1H), 6.85 (br, 1H), 6.78 (d, *J* = 9.2 Hz, 1H), 6.33 (br, 1H), 3.87-3.75 (m, 6H), 3.65-3.52 (m, 2H), 2.57-2.52 (m, 1H), 2.33 (s, 3H), 2.26 (s, 3H), 1.61 (d, *J* = 8.4 Hz, 1H).

**Separation Method:**

Except that Compound 28 of Example 6 was separated and purified using Biotage MPLC, Prep-HPLC purification of compounds of other examples was carried out using Aglient 1260, Waters 2489 or GeLai 3500 HPLC at a column temperature of 25°C and a detection wavelength of 214 nm, 254 nm or 280 nm respectively, with other separation conditions shown in the table below:

| Example | Compound | Separation column model | Mobile phase and gradient | Flow rate (mL/min) |
|---|---|---|---|---|
| **1** | 9 | Waters SunFire Prep $C_{18}$ OBD (19 mm×150 mm×5.0 μm) | A: MeCN; B: 0.05% aqueous solution of ammonium bicarbonate<br>Gradient: 0 min 40% A, 60% B 16 min 90% A, 10% B | 28.0 |
| **2** | 14 | Waters SunFire Prep $C_{18}$ OBD (19 mm×150 mm×5.0 μm) | A: MeCN; B: 0.05% aqueous solution of ammonium bicarbonate<br>Gradient: 0 min 30% A, 70% B 16 min 90% A, 10% B | 28.0 |
| **3** | 15 | Waters SunFire Prep $C_{18}$ OBD (19 mm×150 mm×5.0 μm) | A: MeCN; B: 0.05% aqueous solution of ammonium bicarbonate<br>Gradient: 0-2 min 20% A, 80% B 16 min 80% A, 20% B | 24.0 |
| **5** | 24 | Waters SunFire Prep $C_{18}$ OBD (19 mm×150 mm×5.0 μm) | A: MeCN; B: 0.05% aqueous solution of formic acid<br>Gradient: 0 min: 10% A, 90% B 16 min: 60% A, 40% B | 30.0 |

(continued)

| Example | Compound | Separation column model | Mobile phase and gradient | Flow rate (mL/min) |
|---|---|---|---|---|
| **6** | 28 | C18 spherical 20-35 μm 100A , 4g | A: MeCN; B: 0.05% aqueous solution of ammonium bicarbonate<br>Gradient: 0 min: 0% A, 100% B 20 min: 100% A, 0% B | 16.0 |
| **7** | 29 | Waters SunFire Prep $C_{18}$ OBD (19 mm×150 mm×5.0 μm) | A: MeCN; B: 0.05% aqueous solution of ammonium bicarbonate<br>Gradient: 0-4 min 30% A, 70% B 24 min 90% A, 10% B | 28.0 |
| **8** | 30 | Waters SunFire Prep C18 OBD (19 mm×150 mm×5.0 μm) | A: MeCN; B: 0.05% aqueous solution of formic acid<br>Gradient: 0 min: 10% A, 90% B 16.0 min: 70% A, 30% B | 30.0 |
| **9** | 31 | Waters XBridge Prep C18 OBD (19 mm×150 mm×5.0 μm) | A: MeCN; B: 0.05% aqueous solution of ammonium bicarbonate<br>Gradient: 0 min 30% A, 70% B 16 min: 90% A, 10% B | 28.0 |

## Biological Evaluation

### Experimental Example 1: RET Inhibition Experiment

Experimental method: According to the instructions of HTRF KinEASE-TK kit (Cisbio), the inhibitory effect of the compound of the present disclosure on the activity of drug-resistant mutant RET enzymes (RET-G810R, RET-G810S and RET-G810C) was determined. The three drug-resistant mutant RET enzymes were each pre-incubated with different concentrations of test compounds (in the inhibition rate test of the compound: for RET-G810R and RET-G810C, the compound concentration was 300 and 1,000 nM; for RET-G810S, the compound concentration was 30 and 300 nM. In the $IC_{50}$ test of the compound: for RET-G810R and RET-G810C, the compound concentration was 1-10,000 nM; for RET-G810S, the compound concentration was 0.1-10,000 nM, 9 concentrations in each case). After pre-incubation for 30 min at room temperature, the substrate and adenosine triphosphate (ATP) were added to start the reaction. For RET-G810R and RET-G810S, incubation was carried out at room temperature for 90 min; for RET-G810C, incubation was carried out at room temperature for 120 min. TK antibody-cryptate and streptavidin-XL665 were added, and the test was performed after incubation at room temperature for 60 min. With the solvent group (DMSO) as the negative control and the buffer group (without RET enzyme) as the blank control, the relative inhibitory activity percentage (i.e. inhibition rate) of compounds with different concentrations was calculated as per the following formula:

Relative inhibitory activity percentage = 1 - (compound group of different concentrations - blank control) / (negative control - blank control) * 100%

The relative inhibitory activity percentages of the compounds with different concentrations were plotted with respect to the compound concentrations, and the curve was fitted according to a four-parameter model to calculate the $IC_{50}$ value as per the following formula:

$$y = min + (max - min) / (1 + (x/IC_{50})^{(-Hillslope)})$$

where y is the relative inhibitory activity percentage, max is the maximum value of the fitted curve, min is the minimum

value of the fitted curve, x is the logarithmic concentration of the compound, and Hill slope is the slope of the curve.

The experimental results are shown in Table 1-7.

**Table 1.** Inhibition rates of the compounds of the present disclosure at a concentration of 300 nM on the activity of mutant RET-G810R enzyme

| Compound No. | Inhibition rate on RET-G810R (%) |
|---|---|
| 2 | 70.41±4.57 |
| 3 | 72.49±3.15 |
| 4 | 78.98±0.24 |
| 6 | 77.16±3.87 |
| 9 | 78.59±4.50 |
| 10 | 72.78±1.58 |
| 12 | 69.44±0.31 |

**Table 2**. $IC_{50}$ (nM) for inhibition of RET-G810R enzyme by the compounds of the present disclosure

| Compound No. | $IC_{50}$ (nM) for inhibition of RET-G810R |
|---|---|
| 5 | 185.30 |
| 18 | 132.40 |

As can be seen from Table 1 and Table 2, the compounds of the present disclosure showed significant inhibition on RET-G810R enzyme.

**Table 3.** Inhibition rates of the compounds of the present disclosure at a concentration of 300 nM on the activity of mutant RET-G810S enzyme

| Compound No. | Inhibition rate on RET- |
|---|---|
| | G810S (%) |
| 4 | 98.89±0.18 |
| 5 | 97.89±0.57 |
| 6 | 83.01±1.42 |
| 7 | 91.80±0.98 |
| 8 | 83.73±3.16 |
| 9 | 97.64±0.19 |
| 10 | 88.46±2.23 |
| 11 | 90.87±1.13 |
| 12 | 99.19±0.55 |
| 13 | 97.21±1.01 |
| 14 | 92.56±0.08 |
| 15 | 97.22±0.39 |
| 16 | 85.27±0.37 |
| 17 | 90.64±0.68 |
| 19 | 80.98±7.32 |
| 20 | 96.67±0.03 |

(continued)

| Compound No. | Inhibition rate on RET- |
|---|---|
| | G810S (%) |
| 25 | 97.26±0.72 |
| 26 | 97.83±0.32 |
| 28 | 90.81±1.24 |
| 29 | 93.21±0.91 |
| 30-A | 99.01±0.15 |
| 31 | 99.69±0.14 |

**Table 4.** $IC_{50}$ (nM) for inhibition of RET-G810S enzyme by the compounds of the present disclosure

| Compound No. | $IC_{50}$ (nM) for inhibition of RET-G810S |
|---|---|
| 18 | 15.94 |
| 21 | 6.61 |
| 24 | 57.21 |
| 27 | 23.11 |

As can be seen from Table 3 and Table 4, the compounds of the present disclosure showed significant inhibition on RET-G810S enzyme.

**Table 5.** Inhibition rates of the compounds of the present disclosure at a concentration of 300 nM on the activity of mutant RET-G810C enzyme

| Compound No. | Inhibition rate on RET-G810C (%) |
|---|---|
| 12 | 69.10±5.56 |
| 19 | 82.80±3.66 |
| 20 | 86.86±1.73 |
| 22 | 84.26±6.82 |
| 27 | 82.66±2.47 |
| 31 | 74.13±1.28 |

**Table 6.** $IC_{50}$ (nM) for inhibition of RET-G810C enzyme by the compounds of the present disclosure

| Compound No. | $IC_{50}$ (nM) for inhibition of RET-G810C |
|---|---|
| 18 | 36.34 |
| 21 | 172.90 |
| 30-A | 124.90 |

As shown in Table 5 and Table 6, the compounds of the present disclosure showed significant inhibition on RET-G810C enzyme.

**Table 7.** $IC_{50}$ (nM) for inhibition of RET mutant enzymes by the compounds of the present disclosure

| Compound No. | $IC_{50}$ (nM) for inhibition of RET-G810R | $IC_{50}$ (nM) for inhibition of RET-G810S | $IC_{50}$ (nM) for inhibition of RET-G810C |
|---|---|---|---|
| BLU-667 | 168.45±0.45 | 1.77±0.27 | 48.81±3.82 |
| LOXO-292 | 73.97±16.40 | 5.66±0.22 | 151.30±30.70 |
| 1-A | 19.62±0.67 | 2.69±0.56 | 53.16±10.67 |

As can be seen from Table 7, Compound 1-A of the present disclosure remarkably inhibited RET-G810R enzyme, RET-G810S enzyme, and RET-G810C enzyme.

**Example 2: Inhibitory Effect of Compound on Proliferation of Three RET (G810) Mutants**

Experimental purpose: To evaluate the inhibitory effect of the compound on proliferation of three BaF3 cell lines (Ba/F3 KIF5B-$RET^{G810R}$, Ba/F3 KIF5B-$RET^{G810S}$, Ba/F3 KIF5B-$RET^{G810C}$) in vitro.

Experimental method: Three Ba/F3 cell lines (Ba/F3 KIF5B-$RET^{G810R}$, Ba/F3 KIF5B-$RET^{G810S}$, Ba/F3 KIF5B-$RET^{G810C}$) in logarithmic phase were respectively collected and inoculated into 96-well plates, with 2000 cells/95 μl/well. To the 96-well plate, 5 μl of solutions of BLU-667, Loxo-292 and Compound 1-A were added respectively (final concentration: 1.52-10000 nM). In addition, negative control wells (without the test compound) and blank control wells (without cells) were set. The cells were cultured at 37°C and 5% $CO_2$ for 3 days, then Cell Titer Glo reagent was added at 50 μl/well to lyse the cells, the Luminescence signal value was detected by a microplate reader, and the inhibition rate % was calculated.

Inhibition rate % = [1 - (average signal value of compound group - average signal value of blank control) / (average signal value of negative control - average signal value of blank control)] * 100%; the inhibition rate % was fitted using the four-parameter equation "log(agonist) vs response-variable slope (four parameters)" in GraphPad Prism 6.0, and the $IC_{50}$ was obtained by calculation.

Experimental results: See Table 8. The following results show that Compound 1-A is superior to BLU-667 and Loxo-292 in inhibiting the proliferation of Ba/F3 KIF5B-$RET^{G810R}$, Ba/F3 KIF5B-$RET^{G810S}$, and Ba/F3 KIF5B-$RET^{G810C}$ cells.

**Table 8.** Inhibition $IC_{50}$ (nM) of the compounds on the proliferation of the three cell lines

| Cells | BLU-667 | Loxo-292 | 1-A |
|---|---|---|---|
| Ba/F3 KIF5B-$RET^{G810R}$ | 181.50±80.02 | 116.30±1.15 | 26.28±5.76 |
| Ba/F3 KIF5B-$RET^{G810S}$ | 28.96±4.47 | 87.83±0.75 | 20.21±0.35 |
| Ba/F3 KIF5B-$RET^{G810C}$ | 146.40±3.40 | 477.00±4.40 | 65.30±1.00 |

**Example 3: Efficacy Test of Compound in Mice**

Experimental purpose: To study the in vivo efficacy of the compound in BALB/c Nude mouse model with Ba/F3 KIF5B-$RET^{G810R}$ subcutaneous tumour.

Drug preparation: Compound 1-A, BLU-667 and Loxo-292 were respectively mixed with 0.5% methyl cellulose (MC) as the solvent to prepare homogenous suspensions for administration (PO (IG), BID).

Tumour measurement: The tumour diameter was measured with a vernier caliper twice a week. The formula for calculation of tumour volume is: $V = 0.5 \times a \times b^2$, where a and b represent the long and short diameters of the tumour respectively. The antitumour effect of the compound was evaluated by TGI (%). TGI (%) represents the tumour growth inhibition rate. TGI (%) = [(1-(average tumour volume at the end of administration in the treatment group - average tumour volume at the beginning of administration in the treatment group)]/(average tumour volume at the end of treatment in the solvent control group - average tumour volume at the beginning of treatment in the solvent control group)] × 100%. The results are shown in Table 9 and FIG. 1.

Statistical analysis: Prism Graphpad 8.0 software was used for statistical analysis based on the relative tumour volume at the end of the experiment. The comparison between two groups and multiple groups was analysed by two-way ANOVA, and $p<0.05$ represented significant difference.

Experimental results: No mouse died in the experiment. In the Ba/F3 KIF5B-$RET^{G810R}$ model, the tumour

growth inhibition rate (TGI (%)) of Compound 1-A and the control compounds BLU-667 and Loxo-292 at the same dose of 30 mg/kg was 72.37%, 29.78% and 2.69%, respectively, indicating that the tumour inhibition effect of Compound 1-A is better than that of the control compounds BLU-667 and Loxo-292, with $p<0.05$ in both cases.

**Table 9.** Analysis of efficacy of compound on Ba/F3 KIF5B-RET$^{G810R}$ tumour-bearing mouse model (based on tumour volume)

| **Group** | **Number of mice** | **Dosage regimen** | **$V_{P-1}$ ($mm^3$, mean±SEM)** | **$V_{P12}$ ($mm^3$, mean±SEM)** | **TGI (%)** | ***p* value** (vs solvent group) |
|---|---|---|---|---|---|---|
| 1 | 7 | Solvent group (0.5% MC), PO BID × 12 | 101.26±4.78 | 743.62±91.16 | - | - |
| 2 | 7 | BLU-667, 30 mg/kg, PO, BID × 12 | 101.82±5.93 | 552.89±29.7 | 29.78 | <0.05 |
| 3 | 7 | Loxo-292,30 mg/kg, PO, BID × 12 | 100.47±4.52 | 725.55±54.84 | 2.69 | >0.05 |
| 4 | 7 | 1-A, 30 mg/kg, PO, BID × 12 | 101.08±4.75 | 278.53±51.13 | 72.37 | <0.001 |
| Note: $V_{P-1}$: tumour volume one day before administration; $V_{P12}$: tumour volume on Day 12 of administration. | | | | | | |

The above examples do not limit the embodiment of the present disclosure in any way. In addition to those described herein, various modifications of the present disclosure will be apparent to those skilled in the art based on the foregoing description. Such modifications are also intended to fall within the scope of the appended claims. The references cited in the present disclosure (including all patents, patent applications, journal articles, books, and any other publications) are incorporated herein by reference in their entirety.

## Claims

1. Use of a compound of Formula I, a stereoisomer, tautomer, or mixture of stereoisomer and tautomer thereof, an N-oxide thereof, a pharmaceutically acceptable salt, eutecticum, polymorph, or solvate thereof, or a stable isotope derivative, metabolite, or prodrug thereof in the preparation of a drug for the treatment or adjuvant treatment of a disease or condition associated with an RET drug-resistant mutation (e.g., one or more mutations selected from the group consisting of G810R, G810S, G810C, Y806C, Y806N, and V738A) in an individual:

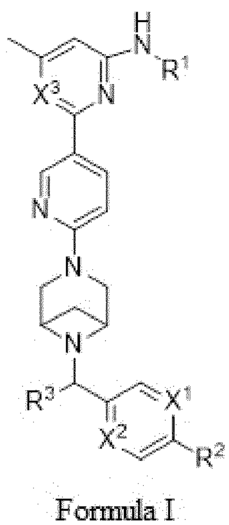

Formula I

wherein:

$R^1$ is selected from 4-10-membered heterocyclyl and 5-10-membered heteroaryl, each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;
$R^2$ is selected from the group consisting of halogen, $C_{1-6}$ alkyl, $C_{1-6}$ haloalkyl, $C_{1-6}$ heteroalkyl, 4-10-membered heterocyclyl and 5-10-membered heteroaryl, wherein the alkyl, heteroalkyl, heterocyclyl and heteroaryl are each optionally substituted with one or more substituents independently selected from the group consisting of hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl, $C_{3-6}$ cycloalkyl and $C_{3-6}$ cycloalkoxy;
$R^3$ is selected from the group consisting of H, halogen, $C_{1-6}$ alkyl, $C_{1-6}$ haloalkyl, $C_{1-4}$ hydroxyalkyl, $C_{1-4}$ heteroalkyl and $C_{3-6}$ cycloalkyl; and
$X^1$, $X^2$ and $X^3$ are each independently selected from CH and N.

**2.** The use according to claim 1, wherein the disease or condition associated with the RET drug-resistant mutation is a drug-resistant disease, preferably a drug-resistant cancer or tumour or irritable bowel syndrome; the cancer or tumour is, for example, an advanced cancer or tumour or a metastatic cancer or tumour; the cancer or tumour is preferably lung cancer (e.g., non-small cell lung cancer), breast cancer, head and neck cancer, rectal cancer, liver cancer, lymphoma, thyroid cancer (e.g., medullary thyroid cancer or papillary thyroid cancer), colon cancer, multiple myeloma, melanoma, glioma, cerebroma or sarcoma;

preferably, the drug-resistant disease is a disease resistant to Selpercatinib and/or Pralsetinib; and
preferably, the drug-resistant disease is non-small cell lung cancer (e.g., RET fusion-positive non-small cell lung cancer), medullary thyroid cancer (e.g., advanced or metastatic medullary thyroid cancer) or thyroid cancer (e.g., advanced or metastatic RET fusion-positive thyroid cancer) resistant to Selpercatinib and/or Pralsetinib.

**3.** Use of the compound of formula I as defined in claim 1, the stereoisomer, tautomer, or mixture of stereoisomer and tautomer thereof, the N-oxide thereof, the pharmaceutically acceptable salt, eutecticum, polymorph, or solvate thereof, or the stable isotope derivative, metabolite, or prodrug thereof in the preparation of a drug for the modulation (e.g., reduction or inhibition) of abnormal RET activity associated with an RET drug-resistant mutation (e.g., one or more mutations selected from the group consisting of G810R, G810S, G810C, Y806C, Y806N, and V738A).

**4.** The use according to any one of claims 1 to 3, wherein the RET drug-resistant mutation is RET solvent front mutation, such as an 810 mutation, and preferably G810 mutation.

**5.** The use according to any one of claims 1 to 4, wherein the RET drug-resistant mutation is one or more mutations selected from the group consisting of G810R, G810S and G810C.

**6.** The use according to claim 5, wherein the RET drug-resistant mutation is G810R mutation.

**7.** The use according to claim 5, wherein the RET drug-resistant mutation is G810S mutation.

**8.** The use according to claim 5, wherein the RET drug-resistant mutation is G810C mutation.

**9.** The use according to any one of claims 1 to 8, wherein $R^1$ is 5-10-membered heteroaryl which is optionally substituted with one or more substituents independently selected from the group consisting of hydroxyl, halogen, CN, $C_{1-4}$ alkyl, $C_{1-4}$ haloalkyl, $C_{3-6}$ cycloalkyl and $C_{3-6}$ cycloalkoxy;

preferably, $R^1$ is 5-6-membered heteroaryl which is optionally substituted with one or more substituents independently selected from the group consisting of halogen, $C_{1-3}$ alkyl and $C_{3-6}$ cycloalkyl;
preferably, $R^1$ is pyrazolyl which is optionally substituted with one or more substituents independently selected from the group consisting of halogen, $C_{1-3}$ alkyl (e.g., methyl) and $C_{3-6}$ cycloalkyl (e.g., cyclopropyl); and
preferably, $R^1$ is pyrazolyl substituted with methyl (e.g., 5-methyl-1H-pyrazol-3-yl or 1-methyl-1H-pyrazol-4-yl) or pyrazolyl substituted with cyclopropyl (e.g., 5-cyclopropyl-1H-pyrazol-3-yl).

**10.** The use according to any one of claims 1 to 9, wherein $R^2$ is selected from the group consisting of halogen (e.g., Cl), $C_{1-6}$ alkyl (e.g., methyl) and 5-6-membered heteroaryl, wherein the alkyl and heteroaryl are each optionally substituted with one or more substituents independently selected from the group consisting of halogen, $C_{1-4}$ alkyl, $C_{1-4}$ hydroxyalkyl, $C_{1-4}$ heteroalkyl, $C_{3-6}$ cycloalkyl and $C_{3-6}$ cycloalkoxy;

preferably, $R^2$ is 5-6-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy;
preferably, $R^2$ is 5-membered heteroaryl which is optionally substituted with one or more substituents independently selected from the group consisting of halogen, $C_{1-4}$ alkyl, $C_{1-4}$ hydroxyalkyl, $C_{3-6}$ cycloalkyl and $C_{3-6}$ cycloalkoxy; and
preferably, $R^2$ is pyrrolyl, pyrazolyl, imidazolyl, furanyl or thiazolyl, which is optionally substituted with one or more substituents independently selected from the group consisting of F, Cl, methyl,

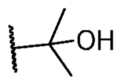,

cyclopropyl and -O-cyclopropyl.

**11.** The use according to any one of claims 1 to 10, wherein $R^3$ is selected from the group consisting of H, $C_{1-6}$ alkyl and $C_{3-6}$ cycloalkyl;

preferably, $R^3$ is selected from H and $C_{1-6}$ alkyl; and
preferably, $R^3$ is H or methyl.

**12.** The use according to any one of claims 1 to 11, wherein:

$X^1$ is CH or N; and preferably, $X^1$ is N;
$X^2$ is CH or N; and preferably, $X^2$ is CH; and/or
$X^3$ is CH or N; and preferably, $X^3$ is N.

**13.** The use according to any one of claims 1 to 12, wherein the compound has a structure represented by Formula I-A or Formula I-B:

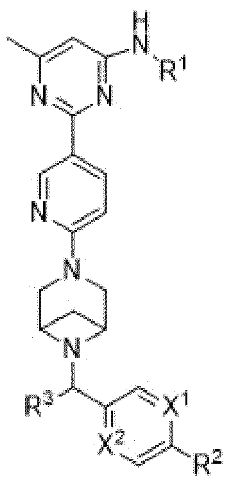

Formula I-A or

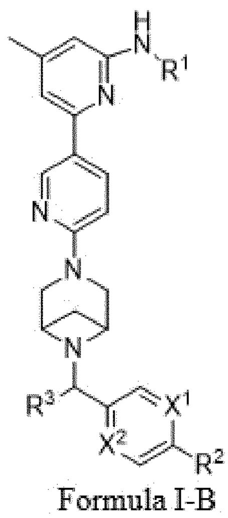

Formula I-B

wherein $R^1$, $R^2$, $R^3$, $X^1$ and $X^2$ are as defined for Formula I.

**14.** The use according to any one of claims 1 to 13, wherein the compound is selected from the group consisting of:

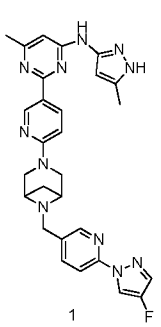
1
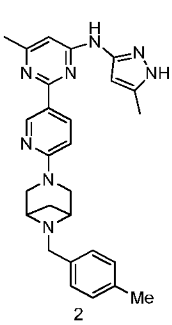
2
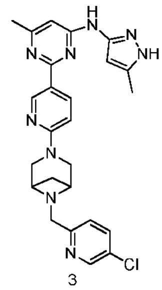
3
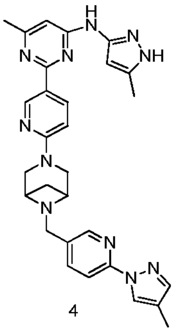
4
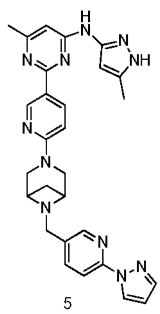
5
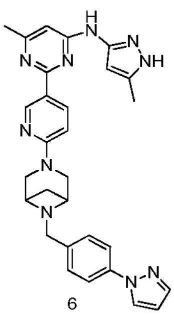
6
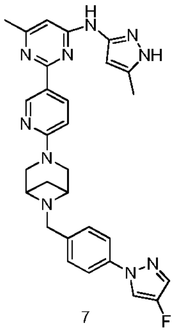
7
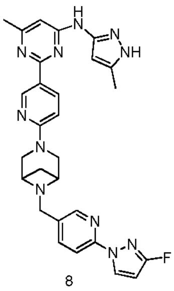
8
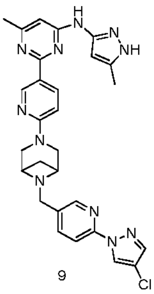
9
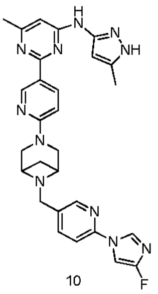
10
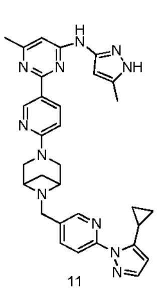
11
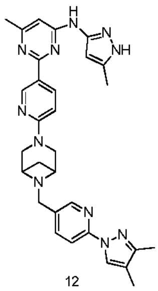
12
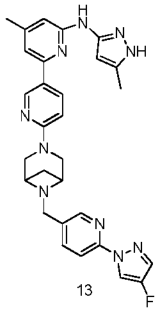
13
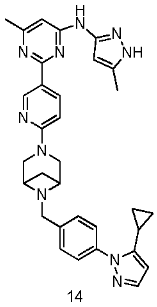
14
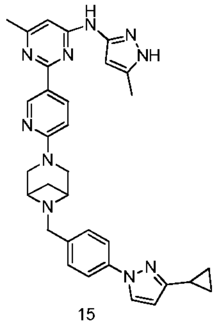
15
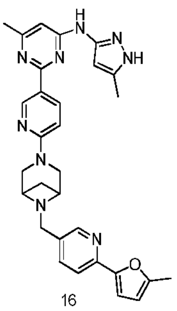
16
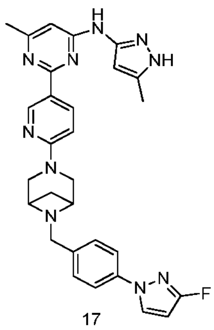
17
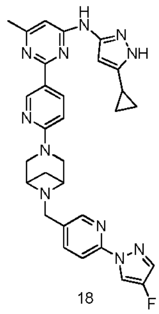
18
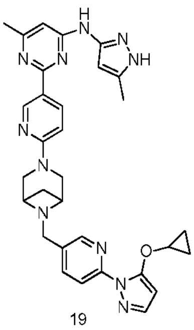
19
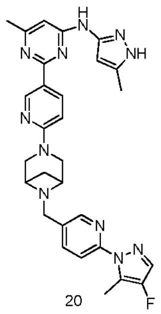
20
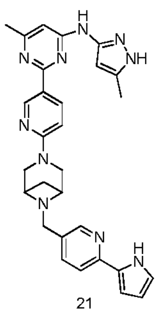
21
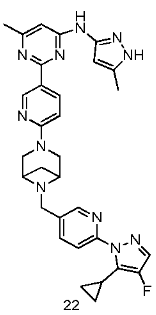
22
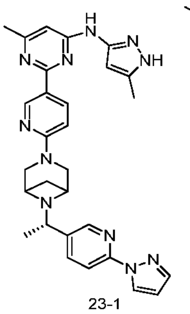
23-1
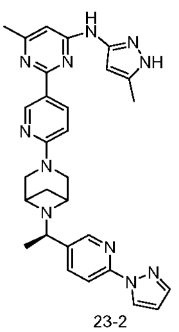
23-2
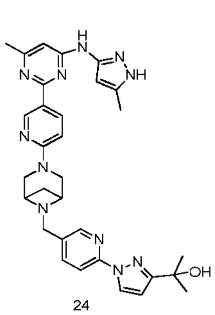
24
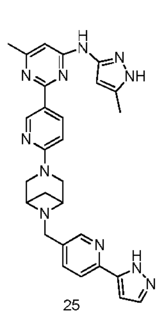
25
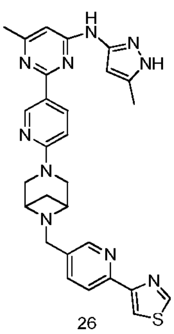
26
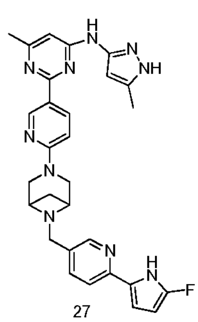
27
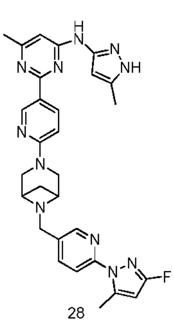
28
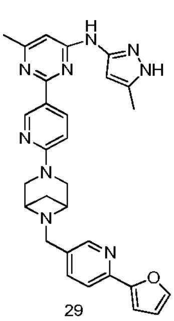
29
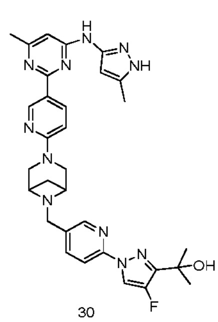
30
and
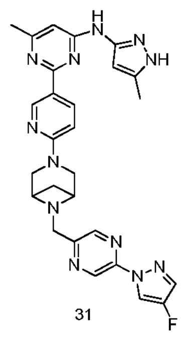
31
.

**15.** The use according to any one of claims 1 to 14, wherein the pharmaceutically acceptable salt is a fumarate.

**16.** The use according to any one of claims 1 to 15, wherein the compound is:

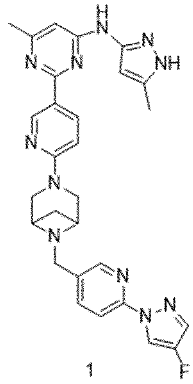

1 .

**17.** The use according to any one of claims 1 to 16, wherein the pharmaceutically acceptable salt of the compound is:

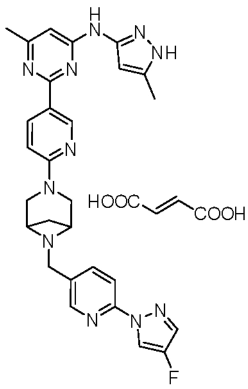

.

## FIGURES

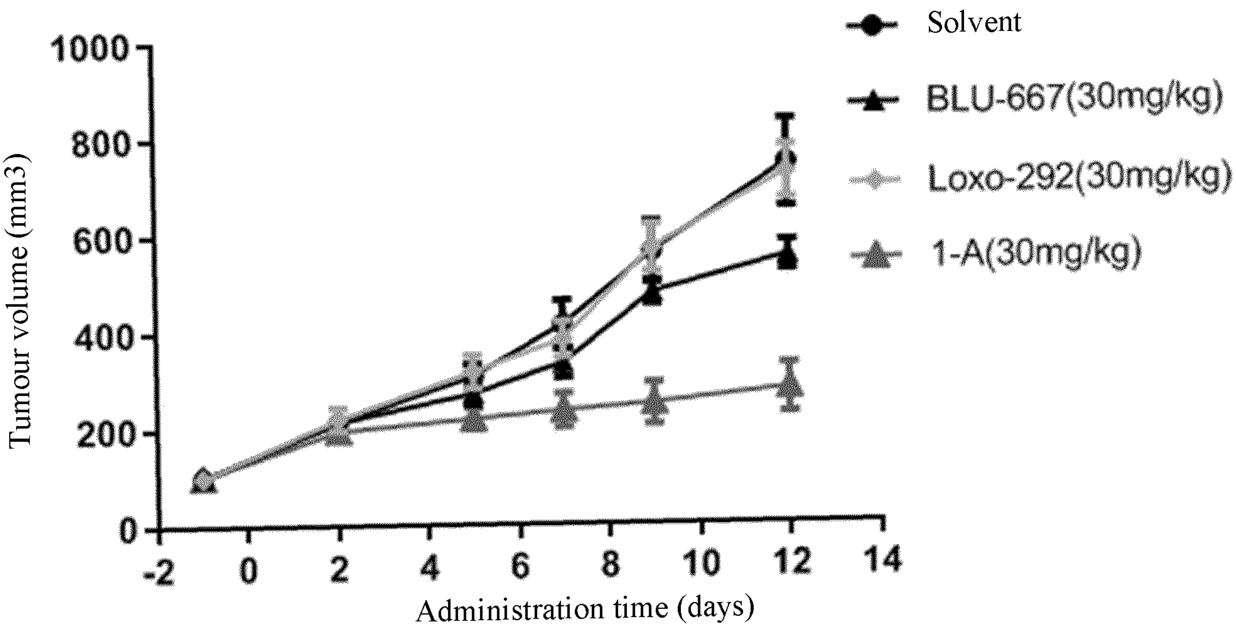


**FIG. 1**

**INTERNATIONAL SEARCH REPORT**

International application No.
**PCT/CN2022/081890**

**A. CLASSIFICATION OF SUBJECT MATTER**

A61K 31/506(2006.01)i; C07D 487/08(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P; C07D

Documentation searched other than minimum documentation to the extent that such documents are included in the fields searched

Electronic data base consulted during the international search (name of data base and, where practicable, search terms used)

WPABSC; CNTXT; CJFD; ENTXTC; VEN; CNKI; ISI WEB OF SCIENCE; REGISTRY; CAPLUS: structural formula search, RET, G810, 耐药, resistance, 癌, 瘤, cancer, tumor 等

**C. DOCUMENTS CONSIDERED TO BE RELEVANT**

| Category* | Citation of document, with indication, where appropriate, of the relevant passages | Relevant to claim No. |
|---|---|---|
| X | WO 2020168939 A1 (SICHUAN KELUN-BIOTECH BIOPHARMACEUTICA CO., LTD.) 27 August 2020 (2020-08-27)<br>entire document, especially claims 8, 9, 11 and 12, embodiments 1, 3 and 4, and tables 1, 2, 3-3 and 3-4 | 1-3, 9-17 |
| Y | WO 2020168939 A1 (SICHUAN KELUN-BIOTECH BIOPHARMACEUTICA CO., LTD.) 27 August 2020 (2020-08-27)<br>entire document, especially claims 8, 9, 11 and 12, embodiments 1, 3 and 4, and tables 1, 2, 3-3 and 3-4 | 1-17 |
| Y | WO 2021023209 A1 (APPLIED PHARMACEUTICAL SCIENCE, INC.) 11 February 2021 (2021-02-11)<br>entire document, especially claims 97 and 107, description, pp. 12-14, 22 and 23, embodiments 1-17 and 24-40, and test examples 1, 3 and 4 | 1-17 |
| A | US 2018022732 A1 (BLUEPRINT MEDICINES CORP.) 25 January 2018 (2018-01-25)<br>entire document | 1-17 |
| A | WO 2020235945 A1 (VORONOI CO., LTD. et al.) 26 November 2020 (2020-11-26)<br>entire document | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 May 2022** | **16 June 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2022/081890

C. DOCUMENTS CONSIDERED TO BE RELEVANT

| Category* | Citation of document, with indication, where appropriate, of the relevant passages | Relevant to claim No. |
|---|---|---|
| A | WO 2012154518 A1 (MERCK SHARP & DOHME et al.) 15 November 2012 (2012-11-15) entire document | 1-17 |

Form PCT/ISA/210 (second sheet) (January 2015)

**INTERNATIONAL SEARCH REPORT**
**Information on patent family members**

International application No.
**PCT/CN2022/081890**

| Patent document cited in search report | | | Publication date (day/month/year) | Patent family member(s) | | | Publication date (day/month/year) |
|---|---|---|---|---|---|---|---|
| WO | 2020168939 | A1 | 27 August 2020 | KR | 20210131314 | A | 02 November 2021 |
| | | | | SG | 11202107848 T | A | 30 August 2021 |
| | | | | US | 2022144847 | A1 | 12 May 2022 |
| | | | | AU | 2020226422 | A1 | 09 September 2021 |
| | | | | EP | 3929198 | A1 | 29 December 2021 |
| | | | | CA | 3130245 | A1 | 27 August 2020 |
| | | | | CO | 2021011023 | A2 | 09 September 2021 |
| | | | | CN | 113316578 | A | 27 August 2021 |
| | | | | IL | 285378 | D0 | 30 September 2021 |
| | | | | JP | 2022521859 | A | 12 April 2022 |
| WO | 2021023209 | A1 | 11 February 2021 | KR | 20220042293 | A | 05 April 2022 |
| | | | | CN | 113490670 | A | 08 October 2021 |
| | | | | EP | 3992197 | A1 | 04 May 2022 |
| US | 2018022732 | A1 | 25 January 2018 | WO | 2018017983 | A1 | 25 January 2018 |
| | | | | US | 10227329 | B2 | 12 March 2019 |
| WO | 2020235945 | A1 | 26 November 2020 | KR | 20200134179 | A | 01 December 2020 |
| | | | | AU | 2020279686 | A1 | 16 December 2021 |
| | | | | IL | 288242 | D0 | 01 January 2022 |
| | | | | SG | 11202112796 Y | A | 30 December 2021 |
| | | | | EP | 3974432 | A1 | 30 March 2022 |
| | | | | CN | 113853375 | A | 28 December 2021 |
| | | | | CA | 3140067 | A1 | 26 November 2020 |
| | | | | BR | 112021023282 | A2 | 04 January 2022 |
| | | | | EA | 202193049 | A1 | 14 February 2022 |
| WO | 2012154518 | A1 | 15 November 2012 | TW | 201249829 | A | 16 December 2012 |
| | | | | EP | 2706852 | A1 | 19 March 2014 |
| | | | | EP | 2706852 | A4 | 05 November 2014 |
| | | | | EP | 2706852 | B1 | 22 August 2018 |
| | | | | US | 2014249130 | A1 | 04 September 2014 |
| | | | | US | 9145391 | B2 | 29 September 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020168939 A1


### Non-patent literature cited in the description


- *Nature Reviews Cancer,* 2014, vol. 14 (3), 173-186
- *Journal of Thoracic Oncology,* 2020, vol. 15 (4), 541-549
- *Annals of oncology: official journal of the European Society for Medical Oncology,* 2021, vol. 32 (2), 261-268
- *Annals of oncology: official journal of the European Society for Medical Oncology,* 2020, vol. 31 (12), 1599-1600
- *Annals of oncology: Official Journal of the European Society for Medical Oncology,* 2020, vol. 31 (12), 1725-1733
- **STAHL ; WERMUTH.** Handbook of Pharmaceutical Salts: Properties, Selection, and Use. Wiley-VCH, 2002
- **T. L. GILCHRIST.** Comprehensive Organic Synthesis. Academic Press, vol. 7, 748-750
- **G. W. H. CHEESEMAN ; E. S. G. WERSTIUK.** Advances in Heterocyclic Chemistry. Academic Press, vol. 22, 390-392
- **T. HIGUCHI ; V. STELLA.** Pro-drugs as Novel Delivery Systems. *ACS Symposium Series,* vol. 14
- **H. BUNDGAARD.** Design of Prodrugs. Elsevier, 1985
- **T. W. GREENE ; P. G. M. WUTS.** Protective Groups in Organic Synthesis. John Wiley & Sons, 1991
- *Remington's Pharmaceutical Sciences,* 1990